Europäisches Patentamt

(19) European Patent Office

Office européen des brevets

(11) Publication number: **0 260 148**
**A2**

## (12) EUROPEAN PATENT APPLICATION

(21) Application number: **87308060.0**

(22) Date of filing: **11.09.87**

(51) Int. Cl.⁴: **C 12 N 15/00**
C 12 N 5/00, C 12 N 9/64

(30) Priority: **12.09.86 US 907185    09.07.87 US 71674**

(43) Date of publication of application:
**16.03.88 Bulletin 88/11**

(84) Designated Contracting States:
**AT BE CH DE ES FR GB GR IT LI LU NL SE**

(71) Applicant: **GENENTECH, INC.**
**460 Point San Bruno Boulevard**
**South San Francisco California 94080 (US)**

(72) Inventor: **Gorman, Cornelia Maxine**
**1122 Church Street**
**San Francisco California 94114 (US)**

(74) Representative: **Armitage, Ian Michael et al**
**MEWBURN ELLIS & CO. 2/3 Cursitor Street**
**London EC4A 1BQ (GB)**

(54) Improved recombinant expression method, vector and transformed cells.

(57) A method for continuous production of a desired heterologous protein comprising constructing an expression vector having a stabilizing sequence downstream of a promoter and upstream of the DNA encoding the desired heterologous protein, transfecting and choosing a particular eukaryotic host cell for said continuous production and culturing the transformed eukaryotic host cell under conditions favorable for continuous production of said desired heterologous protein.

EP 0 260 148 A2

**Description**

## IMPROVED RECOMBINANT EXPRESSION METHOD, VECTOR AND TRANSFORMED CELLS

This invention relates to the application of recombinant DNA technology to prepare vectors capable of expressing desired proteins such that continuous production of the protein can be achieved. Furthermore, the invention relates to the construction of an expression vector capable of generating stable cytoplasmic mRNA so as to give rise to continuous production of the desired protein. In another aspect the invention relates to an expression vector having a specific stabilizing sequence positioned 5' to a DNA encoding a desired protein. The invention further relates to the transfection of eukaryotic cells with such vectors and choosing of a host cell such that continuous production of the desired protein by that cell line is established.

Recombinant technology has recently been applied to eukaryotic cells, specifically mammalian cells were transformed with heterologous DNA coding for a selectable phenotype. Wigler, <M., et al., Cell 11: 223-232 (1977). It has also been shown that eukaryotic cells can be transformed to yield transformants having heterologous DNA integrated into the chromosomal DNA of the eukaryotic cell nucleus.

Successful transformation of eukaryotic cell cultures and expression of DNA sequences coding for a desired protein has been disclosed. See for example, European Patent Publications Nos. 73,659 and 73,656. These successful transformations have utilized vectors to express complimentary DNA (cDNA's) requiring only 5' control signals such as enhancers (Gluzman, Y and Shenk, T. [eds.] Enhancers and Eukaryotic Gene Expression [Cold Spring Harbor Laboratory, 1983]), promoters (Hamer, D. H. et al., Cell 21, 697 [1980]) and 3' polyadenylation sites (Proudfoot, N.J. and Brownlee, G.G., Nature 263, 211 [1976]).

In 1977 it was found that in eukaryotes the cytoplasmic mRNA is not always co-linear with the DNA. DNA sequences encoding proteins were found to be interrupted by stretches of non-coding DNA. There are long stretches of base sequence in the DNA of the gene which do not appear in the final mRNA. It was observed that the primary mRNA transcripts were "spliced" to remove the non-coding sequences, i.e. sequences which do not encode a protein. These non-coding sequences in DNA are generally referred to as introns (formerly referred to as intervening sequences) while the coding sequences are known as exons. RNA polymerase makes a primary transcript of the entire DNA, both exons and introns. This transcript was processed so that the introns were removed while at the same time the exons were all joined together in the correct order. The mechanism producing the foregoing result is referred to as "splicing."

Numerous split or spliced genes have been discovered. In fact, introns exist in virtually all mammalian and vertebrate genes and also in the genes of eukaroytic microorganisms. Introns are not limited to the coding region of a message. For example, one intron was found in the leader region of the plasminogen activator mRNA before the coding sequence in addition to multiple splice sites elsewhere in the gene. Fisher, R. et al., J. Biol. Chem. 260, 1122 (1985). There has been considerable speculation about why introns have evolved and become such a general feature of eukaryotic genes. Crick, F., Science 204, 264, 1979; and, Sharp, P.A., Cell 23, 643-646 (1981).

Given the ubiquity of introns, it is not surprising that splicing was studied in the context of recombinant technology. For example, an SV40 vector was constructed containing a rabbit β-globin cDNA, regions implicated in transcription initiation and termination, splice sites from a multipartite leader sequence located 5' to the β-globin cDNA sequence and a polyadenylation sequence. Mulligan, R.C. et al., Nature 277, 108-114 (1979). This recombinant genome, when infected into monkey kidney cells, was found to produce hybrid mRNAs containing the leader region for the 16S and 19S late RNA and the β-globin coding sequence. This hybrid mRNA produced substantial quantities of the rabbit β-globin polypeptide. Mulligan et al. discuss an experiment in which mutants lacking splicing capability failed to produce discrete mRNAs. Id. at 109.

In an attempt to establish the physiological role that RNA splicing plays in gene expression, Hamer, D.H. and Leder, P., Cell 18, 1299-1302 (1979) manipulated the location and/or presence of a splice site in SV40 recombinants transfected into monkey cells. Hamer and Leder, supra, used one splice site located within the gene encoding the desired protein or used two splice site sequences, one located 5' to and the second within the gene encoding the desired protein. They found that RNA were produced transiently by all of the viruses that retain at least one functional splice junction. They concluded that splicing is a prerequisite for stable RNA formation. Confirming that result, Gruss, P. et al. PNAS (USA), 76, 4317-4321 (1979) found that construction of an SV40 mutant lacking an intervening sequence made no detectable capsid protein. The Gruss paper utilized a multipartite leader having several splice site sequences. The three papers discussed all utilize viral vectors with numerous splice sites at various locations. These viral vectors differ from the nonviral vectors of the instant invention in several respects. First viral introns are located both 5' and 3' to the transcription unit as well as within the coding sequence itself. In the instant invention the stabilizing sequence is located 5' to the gene encoding the desired protein. Viral vectors continue to replicate independent of the host DNA, do not integrate and are lytic. Finally, many viral vectors require early gene function for correct splicing to occur.

These two papers suggest that RNA splicing may be important in a recombinant milieu. However, other studies abandoned splicing to express proteins using only 5' control signals such as enhancers, and promoters and 3' polyadenylation sites. In fact, recent work by Reddy, U.B. et al., Transcriptional Control Mechanisms, J. Cell. Biochem. Suppl. 10D, 154 (1986), found that the inclusion of introns in an expression vector actually reduced the amount of the desired protein expressed. The authors concluded that introns were not an essential part of vectors for the expression of a desired protein. Hall et al. also observed that including an intron was detrimental to protein expression. It was observed that deletion of the acceptor sequence resulted in transient production of unspliced cytoplasmic viral mRNA. Treismann, R. et al. Nature 292, 595-600 (1981). These results support the notion that splicing is not obligatory.

Straightforward expression using standard recombinant control signals such as enhancers, promoters and 3' polyadenylation sites cannot always be achieved. The SV40 promoter without a splice site has been used to direct expression of numerous cDNAs/ (β-galactosidase, Hall, C.V. et al. J. Mol. Applied Genetics 2,; human interferon, Gray, P.W., et al., Nature 295, 503 (1982); hemagglutinin, Gething, et al. Nature 293, 620 (1981); human lecithin-cholesterol acyltransferase, McLean, J. et al., PNAS 33, 2335 (1986); DHFR, Simonsen, C.C. et al., PNAS 80, 2495 (1983); human interleukin-2, Leonard, W.T. et al., Nature 311, 626 (1984); ras-2, Capon, D.J. et al. Nature 304, 1983; src, Snyder, M.A. et al., Cell 32, 891 (1983); and hepatitis B surface antigen, Crowley, C.W. et al., Mol. Cell Biol. 3, 44-55 (1983)). However, no discrete factor VIII message of correct size was detected using an expression vector comprising an SV40 promoter ligated to a cDNA encoding factor VIII transfected into a variety of cells. Transcription of other genes present on the same plasmid, such as DHFR, did produce the correct message. Since the SV40 promoter could express mRNA for certain proteins but not factor VIII, the problem was identified as relating to either transcription/splicing of a mRNA that would permit continuous expression or simply a lack of accumulatioin of the factor VIII message. The former problem is referred to herein as one of the "stability" of the mRNA.

Numerous experiments using various combinations of transcriptional start signals with the cDNA encoding factor VIII were tried. Cells transfected with such vectors were analyzed for factor VIII message by Northern analysis. No discrete message of the correct size was found.

Experiments were also conducted with introns and splice sites present in the vectors. Okayama, H. and Berg. P., Mol. and Cell. Biol. 3(2): 280-289 (1983) utilize a plasmid vector, pcD, containing an SV40 early region promoter, SV40 late region intron comprising one donor site and two acceptor sites, cDNA and a polyadenylation signal. A vector comprising the adenovirus major late promoter and tripartite leader, having three splice sites and a cDNA encoding factor VIII was constructed as described in European Patent Publication No. 160,457. This vector was analyzed and found to be randomly successful in directing expression of full length factor VIII. This could be explained in part by cryptic splicing. The tripartite leader region is spliced onto multiple coding regions to yield a final message. The complexity of the splicing pattern is evident from the fact that 4 primary transcripts can be differentially spliced to yield 14 discrete messages. Nevins, J. and Wilson, M., Nature 290, 113 (1981). The controls for selection of downstream splicing to the coding sequence is not well understood. However, selection of the appropriate polyadenylation site and transcription termination precede the final splicing event and may effect the selection of the 3' splice site. For these reasons and because the information content of the base sequences at exon-intron junctions is relatively small it is not surprising that splicing is sometimes incorrect, i.e. cryptic. Hamer et al., Cell 21, 697-708 (1980) and Mansour et al.. Mol. Cell. Biol. 6, 2684 (1986). Cryptic splicing could explain the random success in expressing full length factor VIII using the adenomajor late promoter and tripartite leader.

Further analyses of vectors containing the adenomajor late promoter was conducted. Adenovectors had been used to express other proteins but with a restricted expression pattern suggesting that the adeno control regions could function in a limited number of cell types. Levine, A.S. et al.. Virol. 11, 672-681 (1973) and Grodzicker, T.J. et al., J. Virol. 9, 559-571 (1976). Vectors were constructed using cDNA's from other proteins such as DHFR or t-PA with the identical 5' and 3' control regions as described in European Patent Publication No. 160,457. Following transfection of these plasmids into Cos, 293, BHK and CHO cells the transfectants were monitored for either t-PA expression by immunoperoxidase staining or DHFR expression using methotrexate. In summary, at no time were any of these adeno late vectors found capable of expressing t-PA or DHFR in any cell types other than 293 or Cos cells. Transient expression of t-PA was reproducibly seen in 293 or COS cells, however, factor VIII expression was random under the identical conditions. These results were confirmed in three papers in which the use of a portion of a viral multipartite leader sequence failed to express the desired protein. In the first paper, Kaufman, R.J. and Sharp, P.A., Mol. Cell. Biol. 2(11), 1304 (1982) constructed a vector containing the adenomajor late promoter, including the first leader and 5' splice donor site of the adenovirus tripartite mRNA leader sequence, adjoined to two 3' splice site acceptor sequences isolated from an immunoglobulin variable-region gene and the DHFR coding region. This vector transfected into DHFR− CHO cells produced a very low frequency of DHFR+ cells. In a second paper Kaufman, R.J. and Sharp, P.A., J. Mol. Biol. 159, 601-621 (1982) described the same plasmid and indicated that expression of DHFR was not obtained. Id. at 606. Wong, G.C. et al., Science 228: 810-815 (1985) use an expression vector having: an SV40 enhancer; the adenovirus major late promoter and tripartite leader sequences; a hybrid intron consisting of a 5' splice site from the first exon of the tripartite leader and a 3' splice site from a mouse immunoglobulin gene; two cDNAs the first encoding a desired protein, colony stimulating factor, and the second DHFR; SV40 polyadenylation sequence; and, VA gene. This polycistronic vector was found to work only transiently, supra at 810, required the presence of VA RNA to increase translatability, supra at 811, and required a second cDNA, that of DHFR, to increase mRNA stability. supra at 811. So while a restricted transient expression capability was seen with adenovirus major late vectors which included the entire tripartite leader for some proteins, certain proteins have additional requirements for successful continuous expression.

A vector was constructed containing a cytomegalovirus promoter and enhancer, a cDNA encoding factor VIII, and a 3' terminating sequence, absent any intron or constructed splice site. Neither transient nor stable expression of factor VIII was observed in any of the cell types tested.

Another vector absent an intron or constructed splice site containing the SV40 early transcriptional sequences including the enhancer and promoter, cDNA encoding factor VIII and the SV40 polyadenylation site produced neither transient nor stable expression.

A vector containing the SV40 promoter and enhancer, the entire adenomajor late tripartite leader i.e. three introns with appropriate donor and acceptor sites, cDNA encoding factor VIII and the 3' hepatitis surface antigen polyadenylation site produced transient expression of factor VIII only in COS cells but no other cell types.

A vector containing the SV40 enhancer and promoter, the first intron of the adenomajor late tripartite leader, an immunoglobulin (Ig) variable region acceptor site, cDNA encoding factor VIII, and the SV40 polyadenylation site expressed factor VIII transiently in COS cells but produced no expression in other cell types.

A vector was constructed containing an SV40 enhancer and promoter, the first donor site and intron of the adenomajor tripartite leader, the consensus sequence for the Ig variable region acceptor sequence, the cDNA encoding factor VIII and the 3' polyadenylation site of the hepatitis surface antigen. This vector failed to provide transient or stable expression of express factor VIII.

Yet another vector was constructed comprising the SV40 enhancer and promoter, cDNA encoding factor VIII, an SV40 small t-antigen intron 3' to the cDNA, complete with donor and acceptor sequence and the SV40 early region polyadenylation site. This vector failed to produce either transient or stable expression of factor VIII in any cell type.

Experiments described herein establish that a stabilizing sequence, either a donor-intron-acceptor sequence or an engineered splice sequence, is necessary for stable expression of certain proteins. Those experiments further establish that location of the stabilizing sequence is important for stable continuous expression. The present invention is directed to the construction and use of vectors having a specific stabilizing sequence positioned 5' to the DNA encoding certain proteins that are difficult to express. The expression vector of the instant invention when transfected into a selected host cell will transfrom that host cell to one that provides continuous production of a desired protein, e.g. factor VIII. The invention is also directed to the choice of an appropriate cell line and transfection of that host cell to establish a cell line for continuous production of the desired protein.

The present invention is based on the discovery that continuous production of some proteins by use of a recombinant expression vector requires a particular arrangement of a stabilizing sequence, located 5' to the DNA encoding the desired protein. Furthermore, the invention relates to a stable cytoplasmic mRNA resulting from use of a stabilizing sequence positioned 5' to a DNA encoding a desired protein. In another aspect the invention is directed to the expression vectors constructed in accord with the foregoing which express the gene encoding the desired heterologous protein.

In still another aspect the invention relates to the choice of an appropriate host cell for transfection with the novel vector of the instant invention. Yet another aspect of the instant invention is the transformation of a host cell to establish a stable cell line for production of the desired heterologous protein.

Figure 1 Construction of a factor VIII expression vector used to establish production cell lines for factor VIII. pF8CIS.

Figure 2 Construction of a factor VIII expression vector used to establish production cell lines for factor VIII. pF8SCIS.

Figure 3 Immunoperoxidase staining of cells following transfection (A) shows expression following transfection with pF8CIS (B) shows expression following transfection with pF8SCIS.

Figure 4 Immunoperoxidase staining of CHO cells transfected with pF8CIS subject to one round of amplification.

Figure 5 Immunoperoxidase staining of CHO cells transfected with pF8CIS and subjected to three rounds of amplification.

Figure 6 Construction of a factor VIII variant expression vector used to establish production cell lines for the factor VIII variant, pF8CIS9080.

Figure 7 Immunoperoxidase staining of the cells transfected with the vector pF8CIS9080 encoding the factor VIII variant or fusion protein.

Figure 8 Immunoperoxidase staining of CHO cells transfected with pF8CIS subjected to continuous amplification.

Figure 9 Construction of an expression vector containing a cDNA encoding factor VIII resistant to proteolytic cleavage by activated protein C. pF8CIS-336E.

Figure 10 Construction of an expression vector containing a cDNA encoding a fusion protein of factor VIII resistant to proteolytic cleavage by activated protein C. pF89080-336E.

Figure 11 SDS-PAGE and Western blot analysis of purified 90kd + 142aa + 80kd fusion. Approximately 8 μg of the 90kd + 142aa + 80kd fusion was resolved by SDS-PAGE. Subsequently the protein was detected by staining with Coomassie blue (A) or transferred to nitrocellulose for Western blot analysis (B). A rabbit polyclonal antibody raised against plasma derived factor VIII was used to detect the 90kd + 142aa + 80kd fusion bound to nitrocellulose.

Figure 12 Thrombin activation of the 90kd + 142aa + 80kd fusion. Approximately 11 μg of the purified 90kd + 142aa + 80kd fusion in 0.05 M Tris, pH 7.4, containing 0.15 M NaCl, 2.5 mM CaCl$_2$ and 5 percent glycerol was incubated with 55 ng of thrombin for 0.1 to 60 minutes at 37°C. At the times indicated an aliquot was removed, diluted 1/2000-1/10,000 fold in 0.05 M Tris, pH 7.4 containing 0.01 percent BSA and assayed by coagulation analysis. SDS buffer was added to the remainder of the sample which was heated to 90°C for 5 min. then subjected to SDS-PAGE (inset).

Figure 13 Binding of 90kd + 142aa + 80kd fusion to vWF is shown. The 90kd + 142aa + 80kd fusion (275 units) in 0.05 M Tris, pH 7.4, 150 nM NaCl$_2$, 2.5 mM CaCl$_2$ and 5 percent glycerol was passed through a vWF-Sepharose column and the column was subsequently washed with three column volumes of the above buffer. The 90kd + 142aa + 80kd fusion was eluted with 0.25 M CaCl$_2$. The vWF-Sepharose column was prepared by coupling pure vWF to Affigel 10 (Bio Rad) according to the manufacturers specifications.

Figure 14 Construction of a prorelaxin expression vector used to establish production cell lines for prorelaxin. pCIHRX.

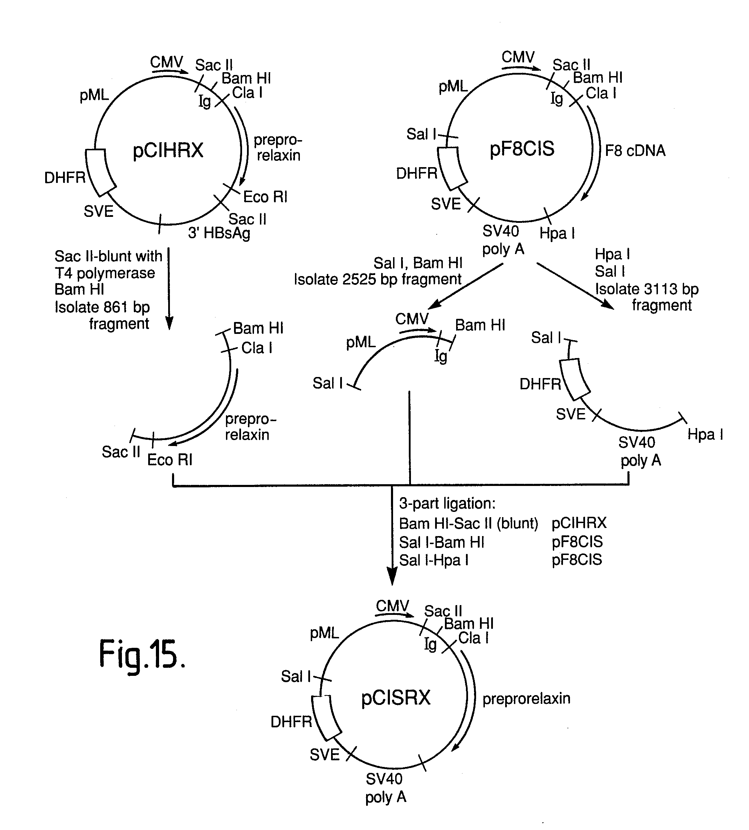

Figure 15 Construction of a prorelaxin expression vector used to establish production cell lines for prorelaxin. pCISRX.

Figure 16 Construction of a t-PA expression vector used to establish production cell lines for t-PA. pCIHt-PA.

Figure 17 Sequence of a portion of pF8CIS. The DNA sequence of the expression vector containing the cytomegalovirus enhancer, promoter (nucleotides 1-732), stabilizing sequence, i.e. splice donor intron sequence, the Ig variable region intron and splice acceptor sequence (nucleotides 733-900).

Figure 18 Sequence of a portion of pF8SCIS. The DNA sequence of the expression vector containing the SV40 enhancer and promoter, (nucleotides 1-360) stabilizing sequence which includes cytomegalovirus donor and intron sequence, the Ig variable region intron and splice acceptor sequence (nucleotides 361-580).

Figure 19 Sequence of a portion of pF8CSSS. The DNA sequence of the expression vector containing the cytomegalovirus enhancer promoter and leader (nucleotides 1-732), stabilizing sequence including the engineered splice donor and acceptor sequence (nucleotides 733-736), the remaining leader.

Figure 20 Constructions of a t-PA expression vector used to establish production cell lines for t-PA. pCISt-PA.

As used herein, "nucleotide sequence" refers to a nucleic acid comprising a series of nucleotides in a 5′ to 3′ phosphate diester linkage which may be either an RNA or a DNA sequence. If a DNA, the nucleotide sequence may be either single or double stranded. Similarly, "DNA sequence" refers to both single and double stranded embodiments.

"Desired heterologous protein" refers to a protein which is desired to be expressed in a host cell, but which the host cell either normally does not produce itself or produces in small amounts, and which is not normally necessary for the cells continued existence. Such a protein includes any molecule having the pre or mature amino acid sequence and amino acid or glycosylation variants (including alleles) capable of exhibiting a biological activity in common with said desired heterologous protein.

"Splicing" refers to the mechanism by which a single functional RNA molecule is produced by the removal of one or more internal stretches of RNA during the processing of the primary transcript. Splicing is believed to begin with the looping out of the intron so that the 5′ end of the intron (referred to as the donor) is juxtaposed to the 3′ end of the intron (referred to as the acceptor). A comparison of the base sequences at intron-exon junctions reveals consensus sequences, with the first two bases at the 5′ end of each intron being GT and the last two bases at the 3′ end being AG.

"Spliced mRNA" refers herein to mRNA produced by either the removal of one or more internal stretches of RNA or by constructing a DNA which when transcribed produces a mRNA having the same properties as a mRNA which had been subject to splicing but from which no nucleotide sequence had in fact been removed.

"Stabilizing sequence" refers to a DNA sequence that gives rise to a spliced mRNA by coding either a splice donor-intron-acceptor sequence or by coding a sequence comprising a full consensus sequence or a part thereof for the donor and acceptor sequence and the appropriate nucleotides at the donor/acceptor junction such that the resulting mRNA resembles functionally a mRNA which had been spliced. The stabilizing sequence is placed in the leader sequence of the gene encoding the desired heterologous protein. "Leader sequence" refers to that region of mRNA that is in the 5′ untranslated region between the CAP site and the AUG translation start signal.

"Consensus sequence" refers herein to the sequences $\overset{C}{\underset{A}{}}$AG/GT$\overset{A}{\underset{G}{}}$ AGT found to occur at the exon-intron boundary (or donor sequence) and ($\overset{T}{\underset{C}{}}$)nN$\overset{C}{\underset{T}{}}$AG/G found to occur at the intron-exon boundary (or acceptor sequence). See Mount, S.M., Nucleic Acids Research 10(2), 459-472 (1982). Analyses of the frequency with which individual bases occur in particular positions yielded a consensus sequence for the donor and acceptor sequences. It is also known that introns begin with GT and end with AG. Breathnach, R. et al., PNAS (USA) 75, 4853-4857 (1978). It is also known that certain multipartite leader sequences in which multiple splicing events occur may require additional factors of early gene function to achieve proper processing. See Babiss, L.E. et al., Mol. and Cell. Biol. 5(10), 2552-2558 (1985). One of ordinary skill in the art using the knowledge of the donor and acceptor consensus sequences, multipartite leader sequences in which multiple splicing events occur requiring early gene function and the consensus splice sequences rule in accord with the instant invention will be able to select a particular stabilizing sequence for a desired protein.

"Control region" refers to specific sequences at the 5′ and 3′ ends of eukaryotic genes which may be involved in the control of either transcription or translation. Virtually all eukaryotic genes have an AT-rich region located approximately 25 to 30 bases upstream from the site where transcription is initiated. Another sequence found 70 to 80 bases upstream from the start of transcription is a CXCAAT region where X may be any nucleotide. At the 3' end of most eukaryotic genes is an AATAAA sequence which may be the signal for addition of the polyadenylation tail to the 3' end of the transcribed mRNA.

"Promoter" refers to the nucleotide segment recognized by RNA polymerase molecules that start RNA synthesis. Promoters controlling transcription from vectors in mammalian host cells may be obtained from various sources for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication. Fiers et al., 1978, "Nature", 273: 113. The immediate early promoter of the human cytomegalovirus is conveniently obtained as a HindIII E restriction fragment. Greenaway, P.J. et al., Gene 18, 355-360 (1982). Of course, promoters from the host cell or related species also are useful herein.

"Enhancer" refers to cis-acting elements of DNA, usually about from 10-300 bp, that act on a promoter to increase its transcription. Transcription of a DNA encoding a desired heterologous protein by higher eukaryotes is increased by inserting an enhancer sequence into the vector. Enhancers are relatively orientation and position independent having been found 5' (Laimins, L. et al., PNAS 78, 993 [1981]) and 3' (Lusky, M.L., et al., Mol. Cell Bio. 3, 1108 [1983]) to the transcription unit, within an intron (Banerji, J.L. et al., Cell 33, 729 [1983]) as well as within the coding sequence itself (Osborne, T.F., et al., Mol. Cell Bio. 4, 1293 [1984]). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding the desired heterologous protein. The 3' untranslated regions also include transcription termination sites.

Expression vectors may contain a selection gene, also termed a selectable marker. A selection gene encodes a protein, sometimes referred to as a secondary protein, necessary for the survival or growth of a host cell transformed with the vector. Examples of suitable selectable markers for mammalian cells are dihydrofolate reductase (DHFR), thymidine kinase or neomycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR– cells and mouse LTK– cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media. Therefore, direct selection of those cells requires cell growth in the absence of supplemental nutrients.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1, 327 (1982), mycophenolic acid, Mulligan, R.C. and Berg, P. Science 209, 1422 (1980) or hygromycin, Sugden, B. et al., Mol. Cell. Biol. 5:410-413(1985). The three examples given above employ bacterial genes under eukaryotic control to convey resistance to the appropriate drug neomycin (G418 or geneticin), xgpt (mycophenolic acid) or hygromycin, respectively. In the following experiments the selective agent of choice is most often G418 geneticin unless specifically referring to CHO DHFR– cells. In this case the direct selection for DHFR production was used.

"Amplification" refers to the increase or replication of an isolated region within a cell's chromosomal DNA. Amplification is achieved using a selection agent e.g. methotrexate (MTX) which inactivates DHFR. Amplification or the making of successive copies of the DHFR gene results in greater amounts of DHFR being produced in the face of greater amounts of MTX. Amplification pressure is applied notwithstanding the presence of endogenous DHFR, by adding ever greater MTX to the media. Amplification of a desired gene can be achieved by cotransfecting a mammalian host cell with a plasmid having a DNA encoding a desired protein and the DHFR or amplification gene so that cointegration can occur. One ensures that the cell requires more DHFR, which requirement is met by replication of the selection gene, by selecting only for cells that can grow in successive rounds of ever-greater MTX concentration. So long as the gene encoding a desired heterologous protein has cointegrated with the amplifiable gene, replication of this gene gives rise to replication of the gene encoding the desired protein. The result is that increased copies of the gene, i.e. an amplified gene, encoding the desired heterologous protein express more of the desired heterologous protein.

Preferred suitable host cells for expressing the vectors of the instant invention encoding the desired heterologous proteins in higher eukaryotes include: monkey kidney CVI line transformed by SV40 (COS-7, ATCC CRL 1651); human embryonic kidney line (293, Graham, F.L. et al. J. Gen Virol. 36, 59 [1977]); baby hamster kidney cells (BHK, ATCC CCL 10); chinese hamster ovary-cells-DHFR (described by Urlaub and Chasin, PNAS (USA) 77, 4216, [1980]); mouse sertoli cells (TM4, Mather, J.P., Biol. Reprod. 23, 243-251 [1980]); monkey kidney cells (CVI ATCC CCL 70); african green monkey kidney cells (VERO-76, ATCC CRL-1587); human cervical carcinoma cells (HELA, ATCC CCL2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); rat hepatoma cells (HTC, M1.54, Baumann, H. et al., J. Cell Biol. 85, 1-8 [1980]); and, TRI cells (Mather, J.P. et al., Annals N.Y. Acad. Sci. 383, 44-68 [1982]).

"Transformation" means introducing DNA into an organism so that the DNA is replicable, either as an extrachromosomal element or by chromosomal integration. Unless otherwise provided, the method used herein for transformation of the host cells is the method of Graham, F. and van der Eb, A., Virology 52, 456-457 (1973).

Host cells may be transformed with the expression vectors of the instant invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan.

"Transfection" refers to the taking up of an expression vector by a host cell whether or not any coding sequences are in fact expressed. Numerous method of transfection are known to the ordinarily skilled artisan, for example, CaPO4 and electroporation. Successful transfection is generally recognized when any indication of the operation of this vector occurs within the host cell. However, in the context of the present invention successful transfection refers to stable continuous expression of a desired heterologous protein by a host culture over numerous generations.

Choosing of the host production cell is achieved by screening for transient expression and then unamplifed expression using the method of the instant invention. Vectors were screened for transient expression to determine which vectors could be used to express a desired heterologous protein. Transient expression provides an indication of whether the particular plasmid that has been taken up functions, i.e., is transcribed and translated to produce the desired protein. During this time the plasmid DNA which has entered the cell is transferred to the nucleus. The DNA is in a nonintegrated state, free within the nucleus. Transcription of the plasmid taken up by the cell occurs during this period. Vectors which were identified as capable of producing the desired heterologous protein transiently were then used to establish a stable continuous production cell. Transient expression refers to a short period (12-72 hrs) following transfection. Following this initial period after transfection the plasmid DNA becomes degraded or diluted by cell division. Random integration within the cell chromatin occurs. Screening the cells after two to three weeks of unamplified expression is an indicia of cells which have retained the recombinant DNA leading to a permanent cell line.

An assay based on immunoperoxidase staining of a transfected cell was developed to assess quickly whether a desired heterologous protein had been expressed. (Gorman, C.M. et al., Cell 42, 519-522 [1985]). Monoclonal antibodies specific for the desired heterologous protein were screened for use in this assay. Host cells containing the vector were stained and compared to parental cell line for screening cells which produce a specific protein. A monoclonal antibody was selected which gave the strongest signal with the least amount of background. Transient transfections were performed to test vectors for the ability to produce a desired protein. Cells (Cos, 293, CHO, BHK, TM4) were transfected using the CaPO4 technique. (Graham and van der Eb modified by Gorman, C.M. et al., Science 221, 551-553 (1983)). We used ten micrograms per milliliter of precipitate of the specific protein vector to be tested. The precipitates were left on the cells for 3-4 hours. Cells were then glycerol shocked for an average of one minute. Thirty-six hours after transfection cells were fixed with acetone-methanol (50:50) and washed with phosphate buffer saline (PBS). Staining was performed using either a monoclonal antibody supernatant undiluted or purified antibody diluted 1:3000 in PBS containing 10% fetal calf serum. This first antibody remained on the cells 2 hours. Plates were placed on a slow shaker during this time. Cells were washed 5 times over a ten minute period. The second antibody used was rabbit anti-mouse IgG (Dakopatts). This was diluted in PBS + fetal calf serum at a dilution of 1:150. A two hour incubation was followed by another series of washes. To develop the peroxidase reagent orthodiansidine was used as a substrate. An ethanol saturated solution of ortho-diansidine was diluted 1:100 in PBS with 1:10,000 dilution of hydrogen peroxide. This substrate was left on the cells for 2 hrs at room temperature or overnight at 4°C.

By this method a wide variety of vectors encoding the desired protein were quickly screened for the ability to direct protein expression.

Coatest Factor VIII was purchased from Helena Laboratories, Beaumont, TX (Cat. No. 5293). The procedure used was essentially that provided by the manufacturer for the "end point method" for samples containing less than five percent protein.

Production cell lines were established using plasmids of the instant invention which were shown to function transiently in a wide variety of cells. Expression vectors were transfected into a number of cell lines. For these transfections a total of 10 μg of DNA/ml precipitate were used. Selection for expression was made possible in these cells using a selectable marker as described above. All cells were transfected with modified CaPA4 technique except BRL cells which were found to be sensitive to calcium. Electroporation was used with these cells. Transfected cells were selected from suitable host cells as previously described.

The protocol used to establish production cell lines relied heavily on the staining method described above. Two days following transfection, cells were subcultured into a selection media. Media was titrated for the proper amount of the particular substance needed for selection. At the same time that cells were transfected to establish a production cell line, a dish of each cell type was assayed for transient expression.

In order to simplify the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences, restriction sites, in the DNA. The various restriction enzymes used herein are commercially available and their reaction conditions, cofactors and other requirements were used as would be known to the ordinarily skilled artisan. For analytical purposes, typically 1 μg of plasmid or DNA fragment is used with about 2 units of enzyme in about 2 μl of buffer solution. For the purpose of isolating DNA fragments for plasmid construction, typically 5 to 10μg of DNA would be digested with 20 to 40 units of enzyme in a larger volume. Appropriate buffers and substrate amounts for particular restriction enzymes are specified by the manufacturer. Incubation times of about one hour at 37°C are ordinarily used, but may vary in accordance with the supplier's instructions. After digestion the reaction was run directly on a gel to isolate the desired fragment.

"Dephosphorylation" refers to the removal of the terminal 5′ phosphates by treatment with bacterial alkaline phosphatase (BAP). This procedure prevents the two restriction cleaved ends of a DNA fragment from "circularizing" or forming a closed loop that would impede insertion of another DNA fragment at the restriction site. Procedures and reagents for dephosphorylation are conventional. Maniatis, T. et al., 1982, Molecular Cloning pp. 133-134. Reactions using BAP are carried out in 50mM Tris at 68°C to suppress the activity of any exonucleases which may be present in the enzyme preparations. Reactions were run for one hour. Following the reaction the DNA fragment is gel purified.

"Oligonucleotides" refers to short length single or double stranded polydeoxynucleotides which are chemically synthesized by known methods and then purified on polyacrylamide gels.

"Ligation" refers to the process of forming phosphodiester bonds between two double stranded nucleic acid fragments (Maniatis, T. et al., Id., p. 146). Unless otherwise provided, ligation may be accomplished using known buffers and conditions with 10 units of T4 DNA ligase ("ligase") per 0.5 μg of approximately equimolar amounts of DNA fragments to be ligated.

"Filling" or "blunting" refers to the procedures by which the single stranded end in the cohesive terminus of a restriction enzyme-cleaved nucleic acid is converted to a double strand. This eliminates the cohesive terminus and forms a blunt end. This process is a versatile tool for converting a restriction cut end that may be cohesive with the ends created by only one or a few other restriction enzymes into a terminus compatible with any blunt-cutting restriction endonuclease or other filled cohesive terminus. Typically, blunting is accomplished by incubating 2-15μg of the target DNA in 10mM $MgCl_2$, 1mM dithiothreitol, 50mM NaCl, 10mM Tris (pH 7.5) buffer at about 37°C in the presence of 8 units of the Klenow fragment of DNA polymerase I and 250 μM of each of the four deoxynucleoside triphosphates. The incubation generally is terminated after 30 min. phenol and chloroform extraction and ethanol precipitation.

"Northern" blotting is a method by which the presence of a cellular mRNA is confirmed by hybridization to a known, labelled oligonucleotide or DNA fragment. For the purposes herein, unless otherwise provided, Northern analysis shall mean electrophoretic separation of the mRNA on 1 percent agarose in the presence of a denaturant (formaldehyde 7%), transfer to nitrocellulose hybridization to the labelled fragment as described by Maniatis, T. et al., Id., p. 202.

The following examples merely illustrate the best mode now known for practicing the invention, but should not be construed to limit the invention. All literature citations herein are expressly incorporated by reference.

Example 1

Expression Vector

Factor VIII

1. Construction of Expression Vectors

The cDNA encoding human factor VIII was used in the construction of plasmids which would direct the expression of factor VIII protein in transfected mammalian cells (Wood, W. et al., Nature [Lond.] 312:330-337 [1984]). Those transformed mammalian cells secreted approximately .14 mU/ml of factor VIII. The instant method provides continuous production of factor VIII with yields significantly greater.

a) pF8CIS

The vector pF8CIS containing the cytomegalovirus enhancer (Boshart, M. et al., Cell 41, 520 [1985]) and promoter (Thomsen, D.R. et al., PNAS 81, 659-663 [1984]), the cytomegalovirus splice donor site and a portion of an intron (Sternberg, R.M. et al. T. of Virol.49, 190-199 [1984]), the Ig variable region intron and splice acceptor site, the cDNA encoding factor VIII and the SV40 polyadenylation site was constructed.

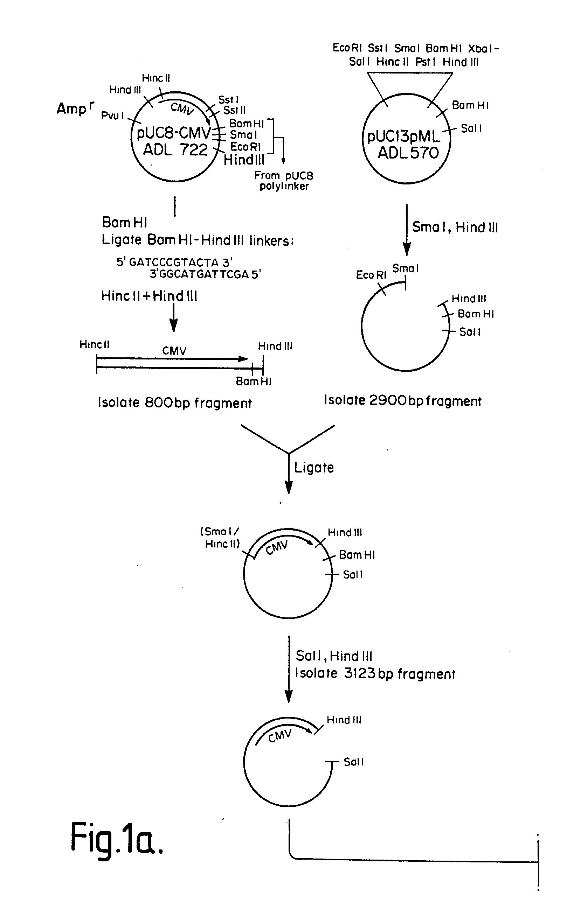

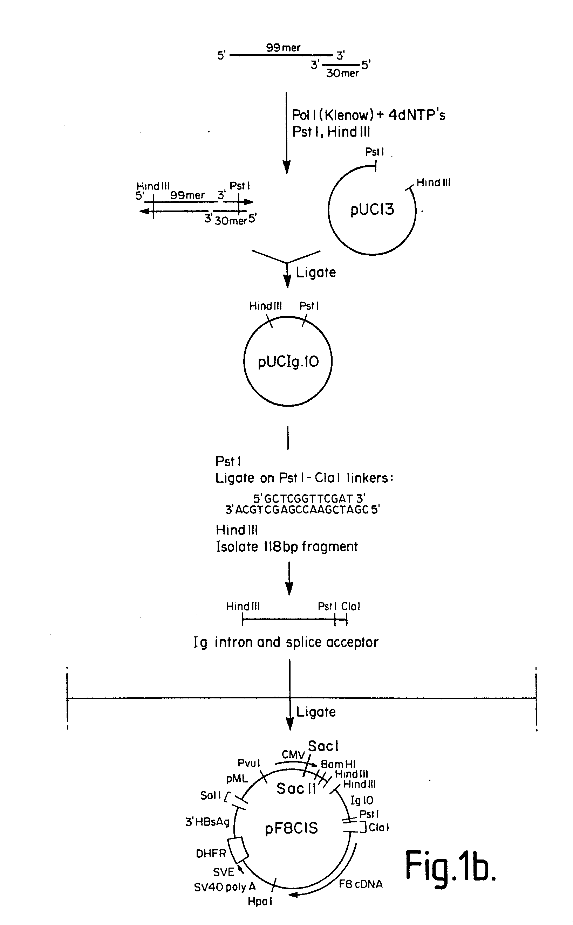

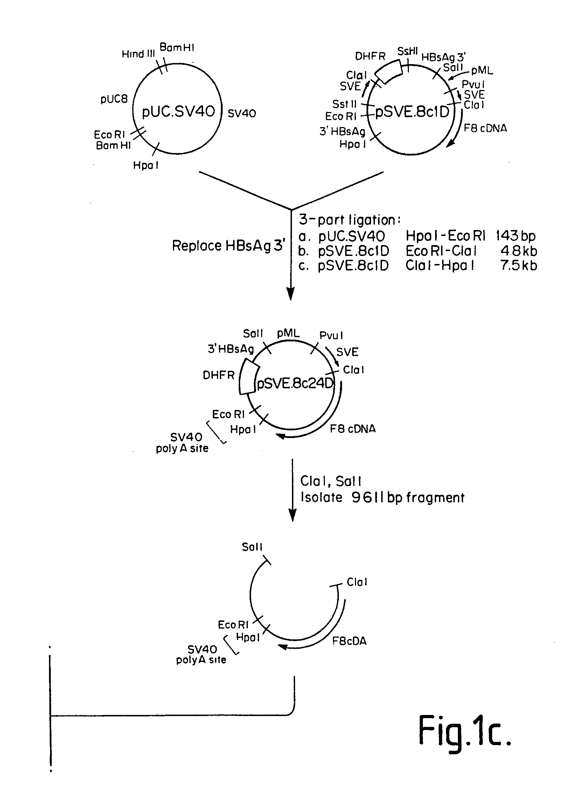

Figure 1 shows the steps for construction of the factor VIII expression vector used to establish production cell lines for factor VIII. The three parts of the construction are detailed below.

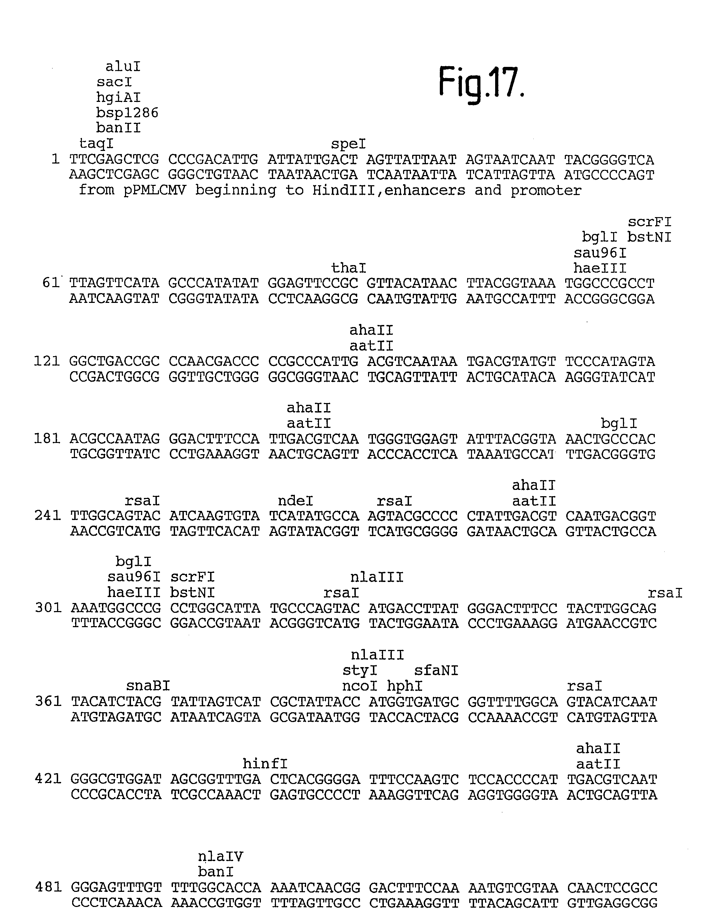

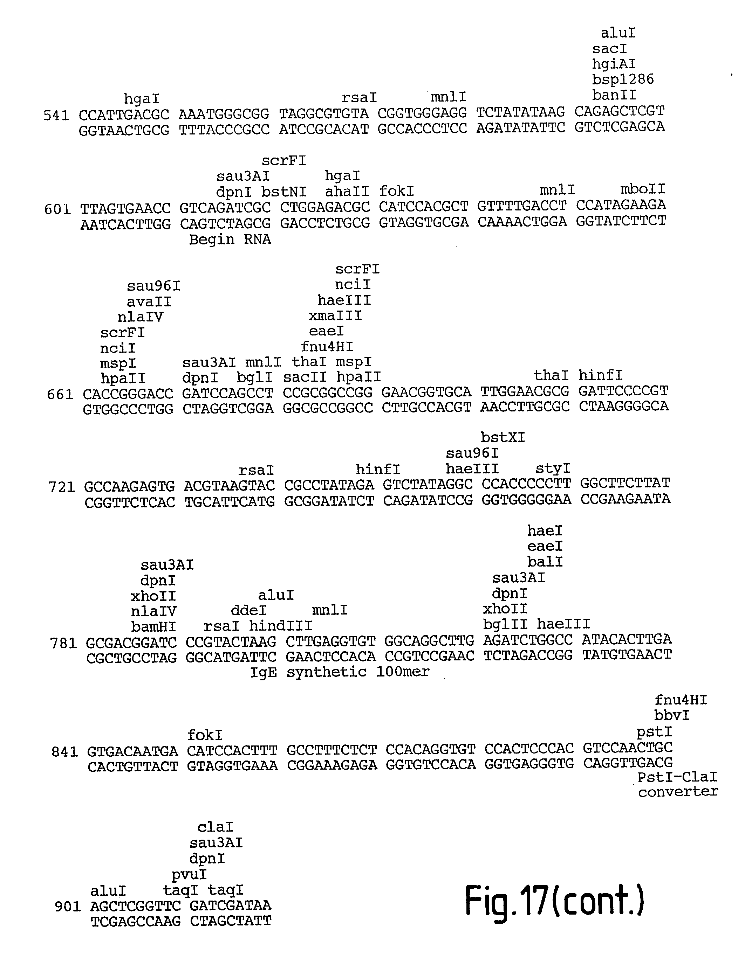

1) The ampicillin resistance marker and replication origin of the final vector was derived from the starting plasmid pUC13pML a variant of the plasmid pML (Lusky, M. and Botchen, M., Nature 293, 79 [1981]). pUC13pML was constructed by transferring the polylinker of pUC13 (Veira, J. and Messing, J., Gene 19:259(1982)) to the EcoRI and HindIII sites of pML. A second starting plasmid pUC8CMV was the source of the CMV enhancer, promoter and splice donor sequence. pUC8CMV was constructed by inserting nucleotides 1 through 732, shown in Figure 17, for the CMV enhancer, promoter and splice donor sequence into the blunted PstI and SphI sites of pUC8. Veira, J. and Messing, J. supra. Synthetic BamHI-HindIII linkers (commercially available from New England Biolabs) were ligated to the cohesive BamHI end creating a HindIII site. Following this ligation a HindIII-HincII digest was performed. This digest yielded a fragment of approximately 800bp which contained the CMV enhancer, promoter and splice donor site. Following gel isolation this 800bp fragment was ligated to a 2900bp piece of pUC13pML. The fragment required for the construction of pF8CIS was obtained by digestion of the above intermediate plasmid with SalI and HindIII. This 3123bp piece contained the resistance marker for ampicillin, the origin of replication from pUC13pML and the control sequences for the CMV including the enhancer, promoter and splice donor site.

2) The Ig variable region intron and splice acceptor sequence was constructed using a synthetic oligomer as shown in the central portion of Figure 1. A 99 mer and a 30 mer were chemically synthesized having the following sequence for the IgG intron and splice acceptor site (Bothwell et al., 1981):

$$1 \quad 5'\text{AGTAGCAAGCTTGACGTGTGGCAGGCTTGA}\ldots$$

$$31 \quad \text{GATCTGGCCATACACTTGAGTGACAATGA}\ldots$$

$$60 \quad \text{CATCCACTTTGCCTTTCTCTCCACAGGT}\ldots$$

$$88 \quad \text{GTCCACTCCCAG}^{3'}$$

$$1 \quad 3'\text{CAGGTGAGGGTGCAGCTTGACGTCGTCGGA}^{5'}$$

DNA polymerase I (Klenow fragment) filled in the synthetic piece and created a double stranded fragment. Wartell, R.M. and W.S. Reznikoff, Gene 9, 307 (1980). This was followed by a double digest of PstI and HindIII. This synthetic linker was cloned into pUC13 (Veira, J. and Messing, J., Gene 19, 259 [1982]) at the PstI and HindIII sites. The clone containing the synthetic oligonucleotide, labelled pUCIg.10, was digested with PstI. A ClaI site was added to this fragment by use of a PstI-ClaI linker. Following digestion with HindIII a 118bp piece containing part of the Ig intron and the Ig variable region splice acceptor was gel isolated.

3) The third part of the construction scheme replaced the hepatitis surface antigen 3' end with the polyadenylation site and transcription termination site of the early region of SV40. A vector, pUC.SV40 containing the SV40 sequences was inserted into pUC8 at the BamHI site described in Viera, J. and Messing, J., supra. pUC.SV40 was then digested with EcoRI and HpaI. A 143bp fragment containing only the SV40 polyadenylation site was gel isolated from this digest. Two additional fragments were gel isolated following digestion of pSVE.8c1D. European Patent Publication No. 150,457. The 4.8 kb fragment generated by EcoRI and ClaI digest contains the SV40-DHFR transcription unit, the origin of replication of pML and the ampicillin resistance marker. The 7.5 kb fragment produced following digestion with ClaI and HpaI contains the cDNA for factor VIII. A three-part ligation yields pSVE.8c24D. This intermediate plasmid was digested by ClaI and SalI to give a 9611bp fragment containing the cDNA for factor VIII with an SV40 polyadenylation and transcription termination sites followed by the SV40 DHFR transcription unit.

The final three part ligation to yield pF8CIS used: a) the 3123bp SalI HindIII fragment containing origin of replication, the ampicillin resistance marker and the CMV enhancer, promoter and splice donor; b) The 118bp HindIII-ClaI fragment containing the Ig intron and splice acceptor; and, c) a 9611bp ClaI-SalI fragment containing the cDNA for factor VIII, SV40 polyadenylation site and the SV40 DHFR transcription unit. A portion of the sequence of the expression vector pF8CIS is shown in Figure 17.

b) pF8CSSS

The vector pF8CSSS containing the cytomegalovirus enhancer and promoter, an engineered stabilizing sequence, the cDNA encoding factor VIII and the SV40 polyadenylation site was constructed. The entire intron region including donor and acceptor sequences was deleted and replaced by an engineered stabilizing sequence. The stabilizing sequence is a synthetic double stranded oligomer having a sequence of the mature mRNA following splicing. The stabilizing sequence was inserted between the unique SacII-ClaI sites of pF8CIS. The sequences of the synthetic oligomers are as follows:

```
SacII
    5'GGCCGGGAACGGTGATTGGAACGCG
    3'CGCCGGCCCTTGCCACTAACCTTGCGC


    5'GATTCCCCGTGCCAAGAGTGACGGTGT
     CTAAGGGGCACGGTTCTCACTGCCACA


      5'CCACTCCCAC GTCCAACTGC
       CGTGAGGGTG CAGGTTGACG


        5'AGCTCCGGTTCGAAT3'
         TCGAGGCCAAGCTTAGC5'
                         ClaI
```

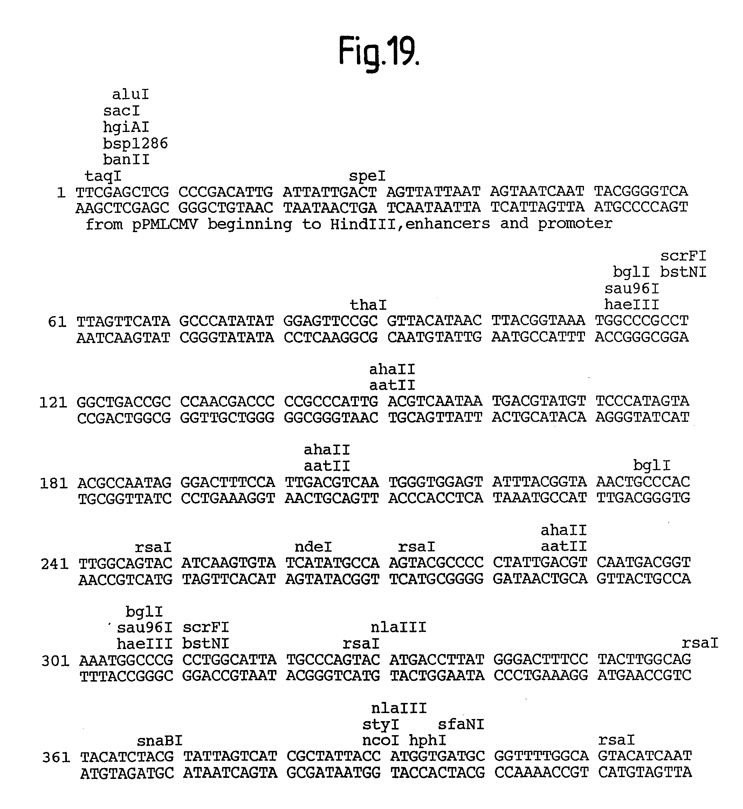

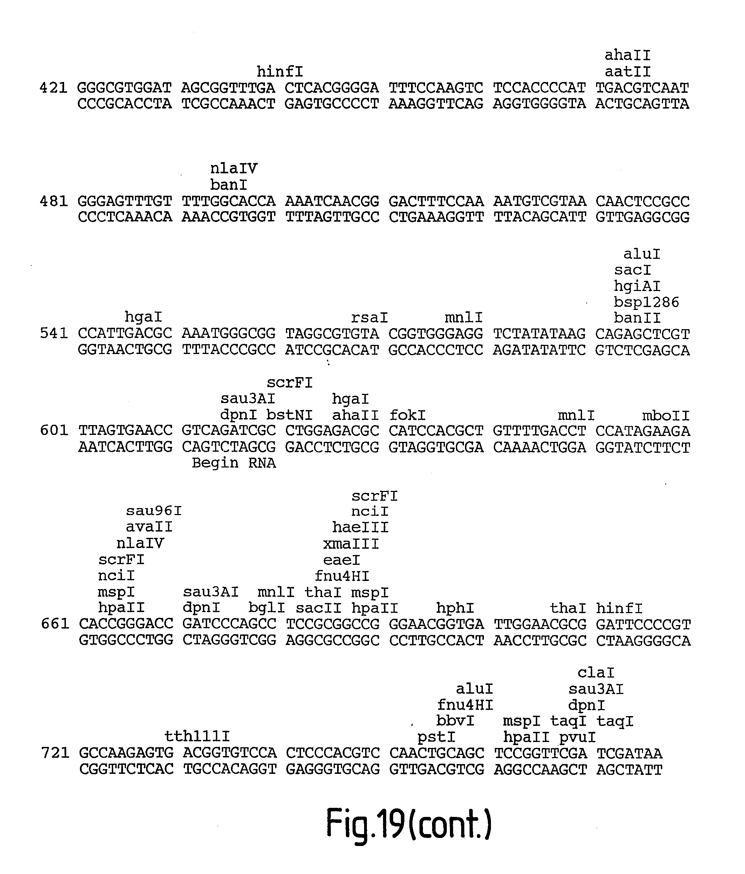

The synthetic oligomers comprise the appropriate nucleotides of the donor and acceptor consensus splice sequences. The juxtaposition of the splice donor sequence to the splice acceptor sequence is indicated by the underline. This vector resembles the pF8CIS vector discussed above except for the deletion of the intron portion and replacement with an engineered stabilizing sequence. This construction eliminates the actual splicing of the noncoding region from recently the transcribed mRNA. A portion of the sequence of the expression vector pF8CSSS containing the engineered stabilizing sequence is shown in Figure 19.

c) pF8SCIS

The vector pF8SCIS containing the SV40 enhancer and promoter, the cytomegalovirus splice donor site and a portion of the intron, the Ig intron and splice acceptor site, the cDNA encoding factor VIII and the SV40 polyadenylation and transcription termination sites were constructed.

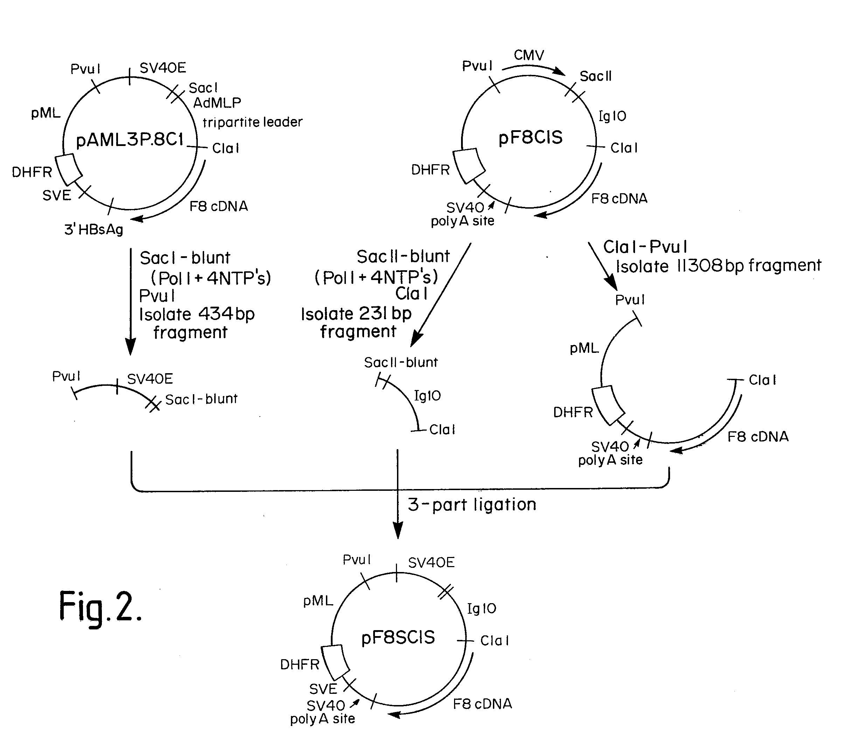

Figure 2 shows the construction of pF8SCIS.

This vector was constructed using a three part ligation. The preparation of each of the three fragment of DNA used in this ligation is described below:

The first fragment contained the SV40 early region promoter and enhancer and one half the ampicillin resistance marker which was obtained from plasmid pML. The starting plasmid for the first of three fragments was pAML3P.8Cl. European Patent Publication No. 160,457. This plasmid was cut with SacI. Using the whole enzyme DNA polymerase I this 3' overhang created by SacI was blunted. Following this reaction the plasmid was cut with PvuI. The desired 434bp fragment was isolated from an acrylamide gel.

The second and third fragments used in this construction were isolated from the plasmid pF8CIS which is described above.

Fragment 2 contained the splice donor from CMV immediate early gene and part of the following intron and the intron and splice acceptor synthetically made as described above. pF8CIS was cut with SacII and the resulting 3' overhang was blunted by the use of DNA polymerase I. This reaction was followed by cleavage with ClaI. Since the sequence surrounding the ClaI site in pF8CIS prevents cleavage if the plasmid is grown in a methylation plus strain, pF8CIS was prepared from dam− strain GM48. Marinus, M.G. and Maris, N.R., Bacteriol. 114, 1143-1150 (1973) and Geier, G.E. and Madrid, P., J. Biol. Chem. 254, 1408-1413 (1979). Since both SacII and ClaI are unique sites in this vector the 231bp fragment was easily isolated from an agarose gel.

The third fragment contains the cDNA for factor VIII, SV40 early region polyadenylation site, a SV40-DHFR transcriptional unit, the origin of replication of pML and half of the ampicillin gene. The 11308bp fragment was prepared by digestion of pF8CIS (dam−) with ClaI and PvuI.

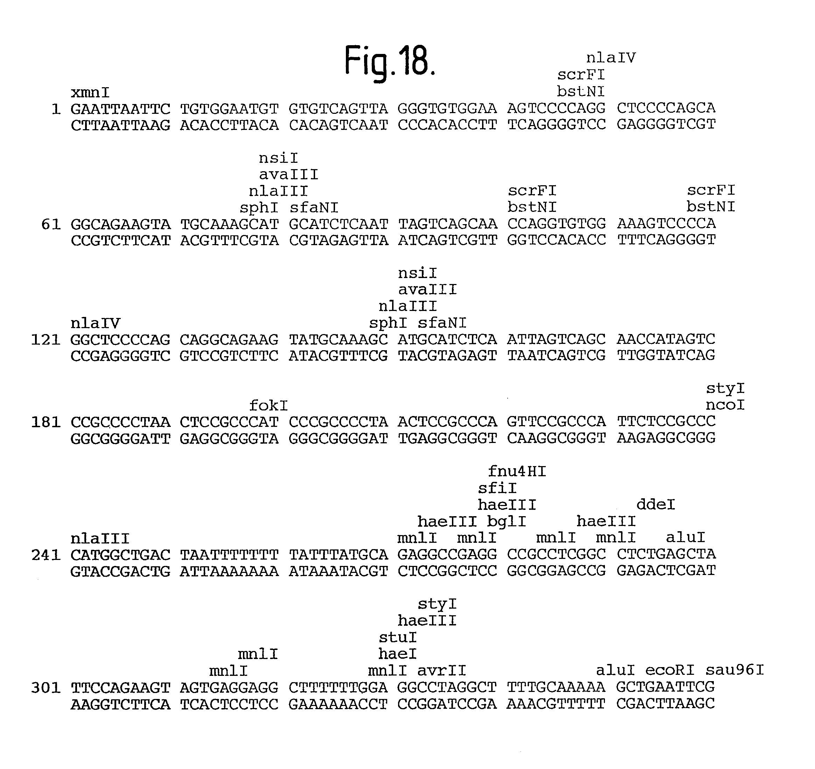

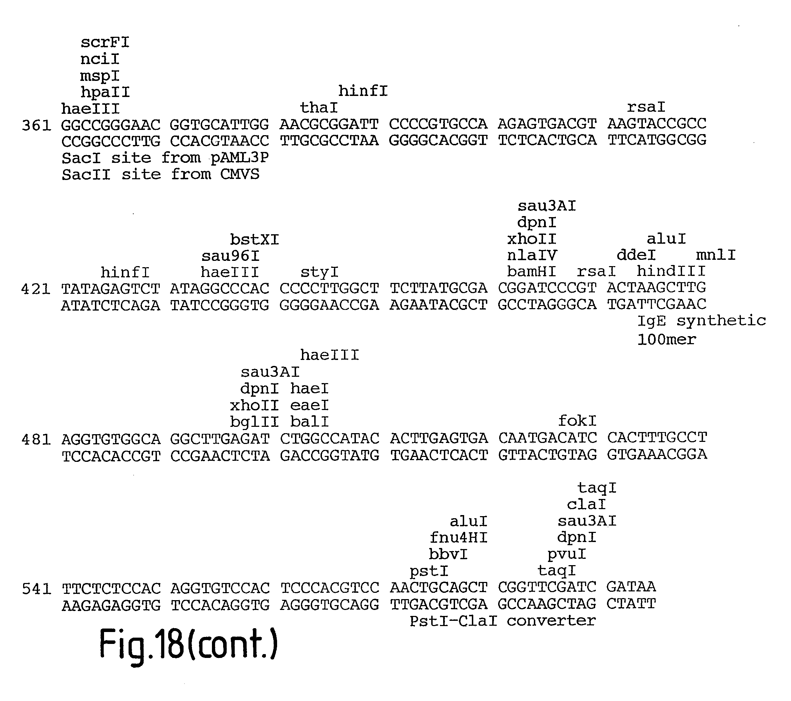

The three part ligation creating pF8SCIS destroys the SacI and SacII sites, maintains the ClaI site and reconstructs the ampᵣgene at the PvuI site. A portion of the nucleotide sequence of the expression vector pF8SCIS is shown in Figure 18.

Example 2

Analysis of Expression

1. Transient Expression

Factor VIII expression was assayed based on immunoperoxidase staining of transfected cells. Gorman et al. Cell 42, 519-526 (1985). This assay was used to test vectors for the expression of factor VIII. Twelve monoclonal antibodies specific for factor VIII were screened for use in this assay. BHK 31A3B cells (European Patent Publication No. 160,457) were stained and compared with parental BHK line to screen cells which produce factor VIII. Monoclonal antibody BH6 was found to give the strongest signal with the least amount of background. Transfections were performed and transient expression of factor VIII was assessed. Cells (Cos, 293, CHO, BHK, TM4) were transfected using the $CaPO_4$ technique. Ten micrograms per milliliter of factor VIII vector precipitate was tested. The precipitates were left on the cells for 3-4 hours. Cells were then glycerol shocked for an average of 1 minute. Thirty-six hours after transfection cells were fixed with acetone-methanol (50:50) and washed with phosphate buffer saline (PBS). Cells were stained using either BH6 supernatant undiluted or purified BH6 antibody diluted 1:3000 in PBS containing 10% fetal calf serum. This first antibody remained on the cells for 2 hours. Plates were placed on a slow shaker during this time. Cells were washed 5 times over a ten minute period. A second antibody of rabbit anti-mouse IgG (Dakopatts) was diluted in PBS + fetal calf serum at a dilution of 1:150. A two hour incubation was followed by another series of washes. Ortho-diansidine (Sigma) was used as a substrate for developing the peroxidase reagent. A ethanol saturated solution of ortho-diansidine was diluted 1:100 in PBS with 1:10,000 dilution of hydrogen peroxide. This substrate was left on the cells for 2 hrs at room temperature or overnight at 4°C.

This method provided a screen for those factor VIII vectors directing factor VIII expression. This method determines transient factor VIII expression. Staining thirty-six hours after transfection provides an indication of whether the vector was transcribed and the mRNA translated.

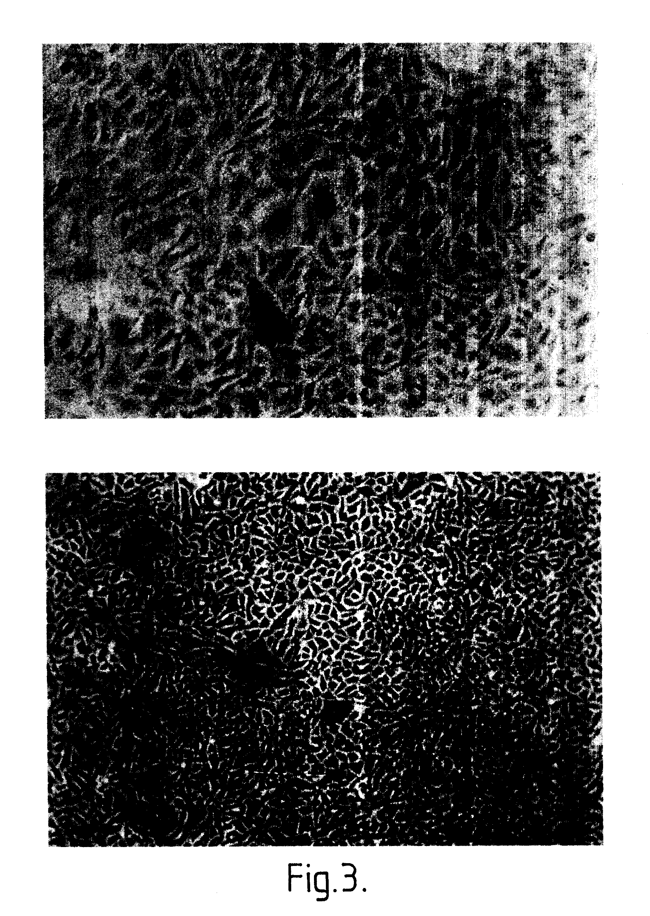

pF8CIS directed transient expression of factor VIII in at least five different cell lines: COS, 293, CHO, TM4 and BHK. Figure 3A shows transient expression of the vector pF8CIS in CHO cells.

pF8SCIS was found to direct transient expression of factor VIII as efficiently as pF8CIS. Figure 3B shows transient expression of the vector pF8SCIS in CHO cells. Since the CMV enhancer and promoter can be completely replaced by the analogous SV40 enhancer and promoter, factor VIII production is not dependent on the specific transcriptional start signal but rather is dependent on other parts of the control region such as the stabilizing sequence site in the vector.

At the same time that cells were transfected to establish a production cell line, a dish of each cell type was assayed for transient expression. Results of the transient expression screen for factor VIII produced two classes of cells: those cell types which stained positively for factor VIII thirty-six hours after transfection (Category 1); and, those cell types having no detectable transient expression of factor VIII (Category 2). The host cells comprising each category are indicated below:

| Category 1 | Category 2 |
|---|---|
| CHO | MDCK |
| 293 | BRL |
| BHK | Hela |
| TM4 | Vero |
| HTC | W138 |
| COS | CV1 |
| HepG2 | |
| TR1 | |

As discussed above deletion of the Ig variable region intron and donor and acceptor sites, while maintaining the other control regions, resulted in elimination of transient expression of factor VIII. From this data at least one splice donor-intron-acceptor sequence appears to be required for expression.

Additional experiments indicate that location of the stabilizing sequence is important. For example location of an intron 3' to the cDNA encoding factor VIII failed to express factor VIII. Vectors which were constructed to include native factor VIII splice sites, i.e. splice sites within the coding region, also proved unsuccessful. The splice donor-acceptor arrangement containing the CMV splice donor sequence and a chimeric intron comprising CMV sequences and the synthesized Ig variable region intron and acceptor is an example of a stabilizing sequence which will lead to the establishment of a cell line providing continuous production of factor VIII.

2. Continuous Production

Production cell lines were established by transfecting the plasmids, containing a stabilizing sequence and shown to function transiently in a wide variety of cells, into a number of cell lines. For these transfections a total of 10 μg of DNA/ml precipitate was used. For transfection of CHO DHFR cells 4 μg of factor VIII plasmid was added to 6 μg salmon sperm DNA, which served as a carrier. Wigler, M. et al., supra. Direct selection for expression of DHFR gene was possible in these cells. All other cell types required cotransfection with a plasmid expressing neomycin gene. Davies, J. and Jenning, A., Am. J. Trop. Med. Hyg. 29(5), 1089-92 (1980). Either pSVENeoBa16 (European Patent Publication No. 160,457) or pRSVneo (Gorman, et al. Science, 221, 551-553 [1983]) were used. For these transfections 4μg factor VIII plasmid 1 μg of neomycin containing plasmid and 5 μg salmon sperm carrier were used. All cells were transfected using modified $CaPO_4$ technique except for BRL as discussed above. Transfected cells included: BHK, CHO-DHFR, CV1, Vero, WI38, 293, TM4, Hela, MDCK, HTC, BRL, TR1 and HepG2.

The protocol used to establish production lines relied heavily on the staining method described above. Two days following transfection cells were subcultured into selection media lacking glycine, hypoxanthine and thymidine for the CHO DHFR cells or G418 containing media. Levels of G418 were titrated for the proper amount needed for selection.

Three to four weeks following the onset of selection, cells were screened for stable expression of factor VIII. A dish of clones was stained to determine the percentage of clones expressing factor VIII. Following this determination twelve clones of each cell type were picked for staining. Clones which scored positive at this time indicating stable expression were then also assayed quantitatively by Coatest assay.

Those cells which failed to demonstrate transient expression did not demonstrate stable expression at this time e.g. Vero, HeLa. Stable expression of factor VIII was observed in two catagories of cells: 1) some cells which expressed factor VIII transiently scored negative during this first round of stable expression e.g. CHO and BHK cells failed to show stable expression levels high enough to stain; and 2) cell lines which stained positively at both transient and unamplified stages e.g. TM4, HTC and 293 which are then referred to as potential production cell lines.

| + | - |
|---|---|
| TM4 | CHO |
| HTC | BHK |
| 293 | Vero |
| | Hela |
| | CV1 |
| | WI38 |
| | BRL |

Low levels of factor VIII in CHO cells were assayed by coatest. Results of the coatest assay were as follows:

### Factor VIII Levels in Unamplified Cells

| Host Cells | $mU/10^4$ cells/day |
|---|---|
| TM4 | 1.8 |
| HTC | 0.5 |
| 293 | 2.5 |
| CHO | <0.15 |
| BHK | <0.15 |

The results of the foregoing assay demonstrate varying levels of factor VIII production within the class of production host cells. The results of immunoperoxidase staining of transfected cells for transient expression at 36-48 hours followed by staining of unamplified cells three to four weeks thereafter was predictive for using a particular cell type as a production cell for factor VIII. If the cells did not stain positively indicating an absence of factor VIII expression at the transient and unamplified levels that host cell is unlikely to serve as a production cell line for factor VIII. This conclusion is supported by the results of staining CHO, BHK, HTC, TM4 and 293 cells for factor VIII expression after rounds of amplification.

Following the first round of amplification (MTX:100nM) clones were again isolated from the foregoing transforming cell types and analyzed for production of factor VIII. Results of factor VIII expression after this first round of amplification were as follows:

0 260 148

<u>Factor VIII levels after One Round of Amplification</u>

| Host Cells | $mU/10^4$ cells/day |
|------------|---------------------|
| TM4 | 3.0 |
| HTC | 2.3 |
| CHO | 0.1 mU/ml |
| BHK | 0.1 mU/ml |

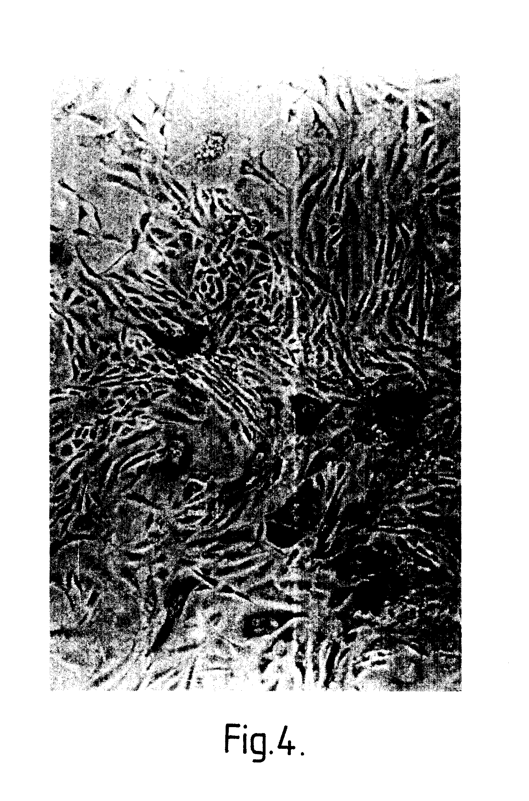

Both clones and mass populations were kept for further amplification. Though factor VIII was monitored transiently in both CHO and BHK cells, few clones were identified after one round of MTX amplification and upon continued passage of cells the low levels of factor VIII were lost. Heterogeneity was seen with these clones. Fig. 4. CHO cells which make factor VIII had a greatly increased doubling time of 45-52 hrs and were overgrown by non-expressing cells in the population which have a doubling time of 28 hrs. Careful study demonstrated that continuous CHO clones expressing factor VIII were difficult to establish. The data presented below shows the frequency of clones expressing factor VIII in DHFR positive CHO clones at both the unamplified level and after one round of amplification. TM4 cells are shown for comparison. The number of clones which stained positive for factor VIII is given as a percentage of the number of stable clones obtained following transfection.

| <u>Cell Type</u> | <u>Vector</u> | <u>Unamplified</u> | 1st Round<br><u>Amplification</u> |
|------------------|---------------|--------------------|-----------------------------------|
| TM4 | pF8CIS | 18% | 77% |
|     | pF8SCIS | 15% | 90% |
| CHO | pF8CIS | 0 | 0.1% |
|     | pF8SCIS | 0 | 0.1% |

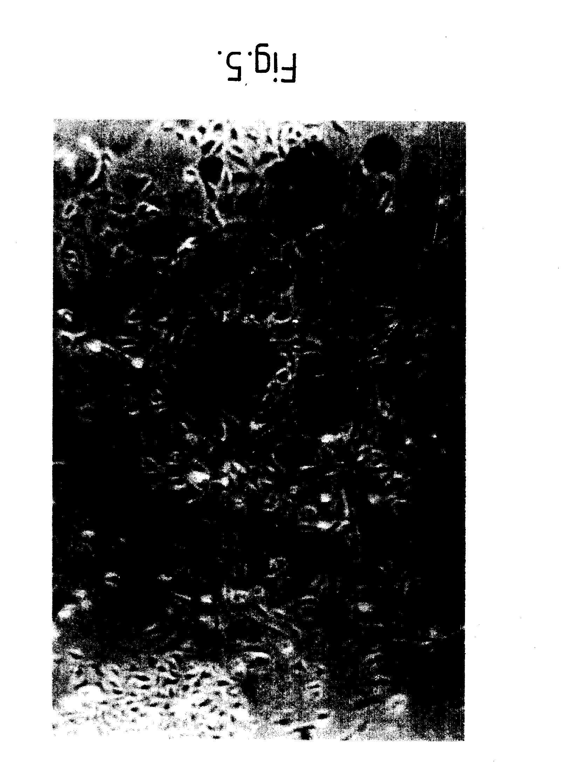

At the second or third round of amplification, usually approximately 1 µM methotrexate, factor VIII was detectable in CHO cells. Even at this high level of amplification, activity of factor VIII was low, 63 mU/ml. Continued amplification did not lead to increased production in CHO cell lines. Morphological analysis of three separately derived CHO lines show the cells staining for factor VIII to be enlarged in size and flattened. Fig. 5. Transformed CHO cells amplified to 10 µM produced no more than 200 mU/ml. These results indicate that the choice of a host cell is an important step in the establishment of a production cell system for factor VIII. Presently TM4, HTC and 293 have been used to establish permanent cell lines providing continuous production of factor VIII, thus qualifying as production cell lines.

Example 3

Coagulant Activity of Factor VIII

The expression and secretion of active factor VIII from TM4 cells was determined by coagulation analysis. Serum free media that had been conditioned for 48 hours by TM4 cells transfected with pF8CIS was assayed for factor VIII. As shown in Table 1 TM4 culture media shortened the clotting time of hemophilic plasma. Most of this coagulant activity was neutralized when TM4 media was preincubated with a polyclonal antibody against human factor VIII.

13

## Table 1

| Sample | Clot Time | Units/ml |
|--------|-----------|----------|
| pd VIII | 46.5 | 0.5 |
| pd VIII + factor VIII antibody | 91.5 | <0.01 |
| TM4 media | 54.2 | 0.36 |
| TM4 media + factor VIII antibody | 70.4 | 0.05 |

Table 1. Secretion of active factor VIII from TM4 cells. TM4 media was either preincubated with 10 µg of a human factor VIII polyclonal antibody or with no addition for 30 minutes at 37°C. Subsequently the media was diluted 1:1 with 0.05 M Tris pH 7.5 containing 0.01% BSA and assayed by coagulation analysis. Purified plasma derived factor VIII (pd VIII) was treated similarly.

Example 4

Expression Vector

Variant Factor VIII

One approach to achieve a more efficient protein is protein engineering. That is, by introducing changes within the gene at the DNA level, variants can be produced in cell culture to allow for specific modification in protein function. Three variants were engineered. The native factor VIII single chain 300,000 dalton protein is cleaved to subunits of 90,000 and 80,000 dalton which in turn are cleaved to the active subunits of 50,000, 43,000 and 73,000 dalton. The B domain between amino acid 742 through 1648 has no defined function. Vehar, G.A. et al., Nature 312, 330-337 (1984). The same cell systems described for expression of the full length recombinant factor VIII protein were used to express the mutant.

pF8CIS9080

The eukaryotic expression vector used to express the factor VIII fusion protein included: the enhancer (Boshart et al., supra), and promoter (Thomsen et al., supra) of the human cytomegalovirus (CMV) immediate early gene; the splice donor sequence located 3' of the transcription initiation site of this gene (Boshart et al., supra, Stenberg et al., supra); and a synthetic splice acceptor site from the mouse immunoglobulin variable region (Bothwell et al., supra). The new coding region is flanked on the 3' end by the SV40 early polyadenylation sequence and transcription termination site (Fiers et al., supra). The vector includes an amplifiable marker, the SV40-DHFR transcription unit.

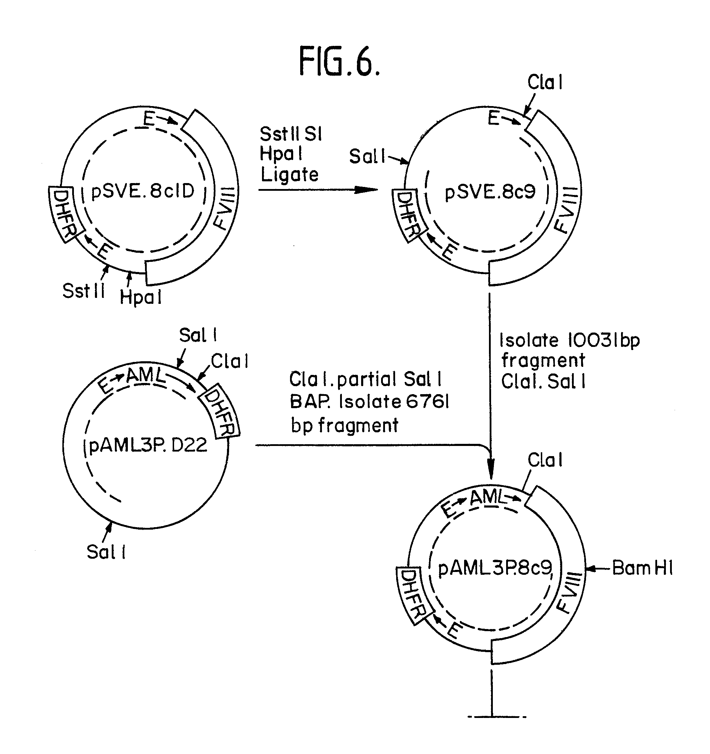

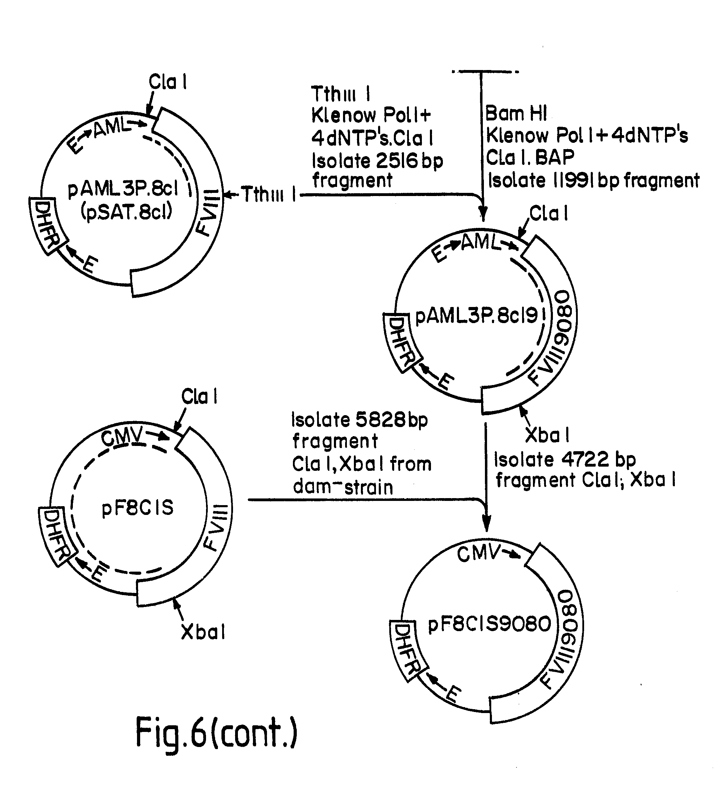

Construction of the expression vector, pF8CIS9080, encoding the factor VIII fusion protein 90kd + 142aa + 80kd is shown in Figure 6. Starting with the plasmid pSVE.8cID (European Patent Publication No. 160,457), a short deletion was made in the 3' untranslated region by cutting with SstII, blunting the cohesive ends with S1, further cleaving with HpaI and religating the two blunt ends to generate pSVE.8c9. This plasmid was cleaved with ClaI and SalI and the 10031bp fragment cloned in the SalI, ClaI. A 6761bp promoter containing fragment of pAML3P.D22 (European Patent Publication No. 160,457). The fusion in the factor VIII gene was made by ligating the filled in Tth111 I and BamHI (amino acid 1563) sites within the factor VIII gene. Figure 6 shows ligation of a 2516bp fragment of pAML3P.8cI (European Patent Publication No. 160,457) and a 11991bp fragment of pAML3P.8c9 to construct pAML3P.8L19 containing the fused region. This fusion was confirmed by DNA sequence. A 4722bp ClaI-XbaI fragment containing the fusion region was cloned into a 5828bp ClaI-XbaI fragment of pF8CIS containing the CMV promoter-enhancer expression vector. The CMV fragment was obtained from a dam– strain of E. coli where methylation does not prevent cutting at the ClaI site.

Example 5

Expression Results

The method described in Example 2 was applied to expression of the factor VIII variant which deleted nucleotides 796 through 1562, pF8CIS9080. The 90kd + 142aa + 80kd fusion protein is expressed at higher levels than the full length protein. However there remains considerable variation between cell types as to the capability of expressing the fusion protein.

The following data demonstrates that the choice of a proper host cell will provide continuous production of the desired fusion protein in commercially useable quantities. TM4 cells transfected with pF8CIS9080 showed both transient and stable expression of the fusion protein. TM4 cells transfected with pF8CIS9080 showed a five-fold increase in the levels of the fusion protein as compared to the full length factor VIII. At 100nM methotrexate pooled clones of the fusion factor VIII yielded 12mU/10⁴ cells/day. HTC cells showed a similar enhancement in expression of the fusion factor VIII as compared to the full length factor VIII.

Expression of the fusion protein factor VIII is quite high in 293 cells as compared to full length factor VIII expression. In 293 cells transformed with the fusion protein vector pF8CIS9080 the unamplified population levels of 85 mU/10⁴ cells/day were routinely achieved. Expression levels of full length factor VIII were lower than the fusion factor VIII yielding 2.5 mU/10⁴ cells/day. Since the control signals are identical in the pF8CIS and pF8CIS9080, the difference in expression levels must lie within the capability of the cell to produce full length message and/or protein.

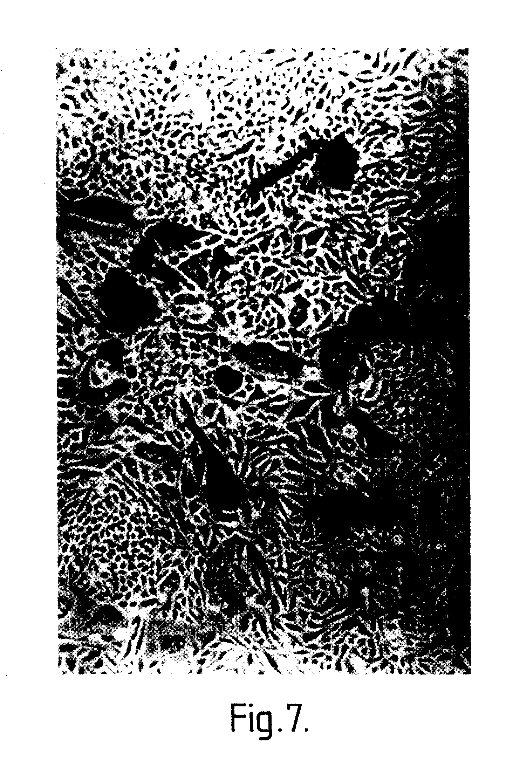

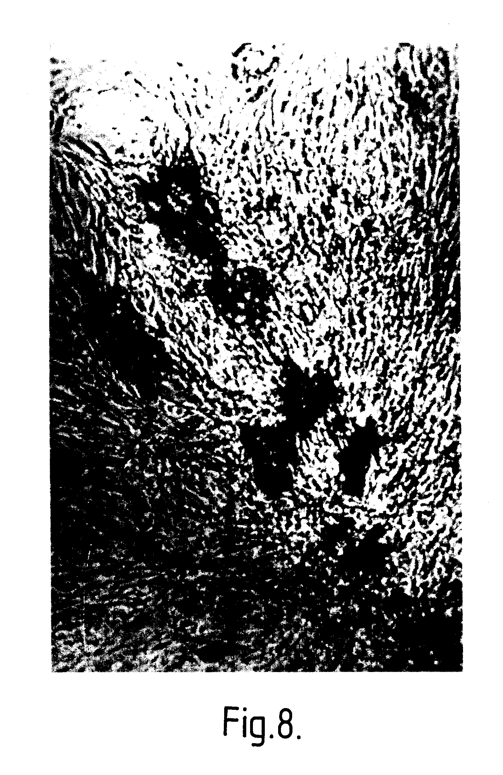

The fusion protein was detected at an earlier point of amplification (100nM) than the full length (1000 nM), however as shown in figure 7 these cells were burdened by the expression of the fusion protein. CHO cells expressing 90kd + 142aa + 80kd at 100 nM produced 0.5 µU/10⁴ cells/day. Continued amplification was difficult due to mixed population seen in figure 8. Clones selected at 1 µM MTX and 5 µM MTX showed no higher expression levels than the 0.1 µM MTX lines. In summary, certain host cells were particularly adept at expression of factor VIII or its variants e.g. TM4. Other host cells were of an intermediate nature in that the variant is expressed while the full length factor VIII is expressed in low levels or not at all e.g. 293 cells. A final group of host cells is unlikely to produce sufficient factor VIII for production, e.g. TRI.

Example 6

Purification and Characterization of Fusion Protein

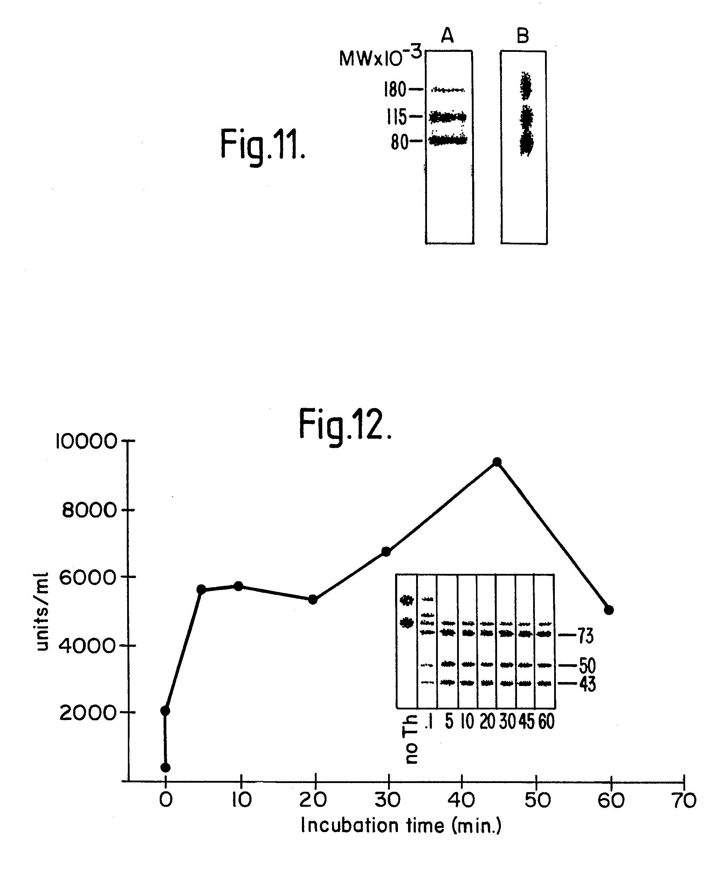

The 90kd + 142aa + 80kd fusion was purified from 293 media using techniques previously described for full length recombinant factor VIII, Eaton, D.E. et al., Biochemistry 25, 505-512 (1986). The purified fusion had a specific activity of 4,000-6,000 units/mg, which is comparable to the specific activity of plasma derived factor VIII. When subjected to SDS-PAGE the fusion resolved into two major bands with $M_r$ of 115,000 and 80,000. A band with a $M_r$ of 180,000 was also seen and probably represents the single chain form of the fusion. The $M_r$ 180,000, 115,000, and 80,000 proteins were all detected by a factor VIII polyclonal antibody in a Western blot (Figure 11).

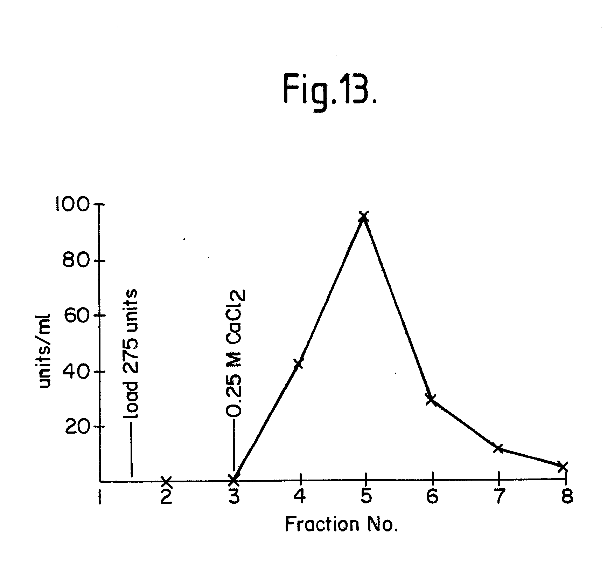

Coagulant activity of the 90kd + 142aa + 80kd fusion was activated 10-20 fold by thrombin (Figure 12). This activation correlated with the generation of subunits with $M_r$ 50,000, 43,000, and 73,000 (Id.). Since factor VIII circulates in plasma bound to von Willebrands factor (vWF), binding of the 90kd + 142aa + 80kd fusion to vWF was also tested. Purified 90kd + 142aa + 80kd fusion that was passed over a vWF-Sepharose column quantitatively bound to the column (Figure 13). Subsequently the fusion was eluted from the column with 0.25 M $CaCl_2$, which is known to dissociate factor VIII-vWF complexes.

The above data show that the 90kd + 142aa + 80kd fusion expressed and secreted from the 293 cells was functionally similar to plasma derived factor VIII. The 90kd + 142aa + 80kd fusion shortened the clotting time of hemophilic plasma and its activity was neutralized by a factor VIII antibody. The fusion was activated and processed by thrombin similarly to plasma derived factor VIII. The 90kd + 142aa + 80kd fusion also bound to vWF immobilized on Sepharaose and was dissociated from vWF under conditions known to dissociate factor VIII-vWF complexes.

Example 7

Coagulant Activity of Fusion Protein

Serum free media that had been conditioned for 48 hrs. by 293 cells transfected with pF8CIS9080 was assayed for factor VIII coagulant activity by coagulation analysis. After the media was diluted 1/50 it was assayed and found to shorten the clotting time of hemophilic plasma from 120 sec. to 58.9 sec., corresponding to 5.5 units/ml of factor VIII coagulant activity. This activity was neutralized by a polyclonal antibody against plasma derived factor VIII (Table 2).

## Table 2

| Sample | Clot Time (seconds) | Units/ml* |
|---|---|---|
| Buffer | 120.0 | <0.1 |
| Diluted 293-media | 58.9 | 5.5 |
| Diluted 293-media Preincubated with factor VIII antibody | 118.0 | <0.1 |
| Undiluted 293-media (parent cell line) | 100.0 | <0.1 |

\* In undiluted media

Table 2. Coagulant activity in 293 media.

Hemophilic plasma (50 μl) was incubated with 50 μl of platelin (General Diagnostics) for 8 min. at 37°C. Subsequently 50 μl of media that had been diluted 1/50 with 0.05 M Tris pH 7.4 buffer containing 0.01 percent BSA was added and incubated 30 sec. $CaCl_2$ (25 mM), 50 μl, was added and the clot time was measured. Media obtained from the parent cell line, which was not transfected, was not diluted. Antibody neutralization experiments were performed by preincubating undiluted media (100 μl) with 10 μg of factor VIII polyclonal antibody for 30 min. at room temp. The media was then diluted and assayed.

### Example 8

Expression Vector of Factor VIII Variant Resistant to Activated Protein C

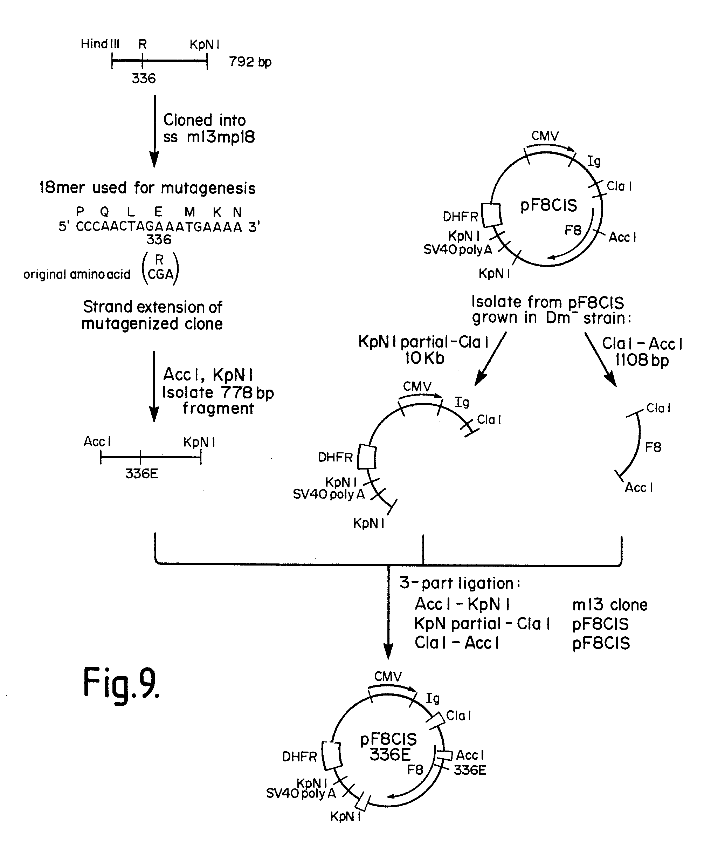

Activated protein C (APC), a plasma protein, has been shown to inactivate human factor VIII by limited proteolysis. One possible site of this inactivation cleavage is at arginine at position 336. The arginine at position 336 can be changed to another amino acid, for example, lysine or glutamic acid. Two vectors, pF8CIS336E and pF8CIS9080-336E, were constructed to determine whether position 336 was a site of inactivation. Using in vitro mutagenesis (Norris, K. et al., Nucleic Acids Research, 11, 5103-5112 [1983]) the arginine at position 336 was mutated to a glutamic acid (Fig. 9). For the mutagenesis a 792 bp HindIII-KpnI fragment from pF8CIS was inserted into the HindIII-KpnI sites of m13. The 18 bp oligomer shown below was used to mutagenize this fragment.

```
        P   Q    L    E    M    K   N
  5'  CC  CAA  CTA  GAA  ATG  AAA  A  3'
                      *
```

Following strand extension the double stranded mutagenized M13 clone was cut with AccI and KpnI. A 778 bp fragment was gel purified. The plasmid pF8CIS was grown in a dam– strain of E. coli, GM48. Due to the sequence of the PstI-ClaI linker shown in figure 1, the ClaI site of pF8CIS will not cut if the plasmid is grown in a methylation plus strain of bacteria as discussed above. Two fragments were isolated from the dam– pF8CIS DNA, a 10kb KpnI partial-ClaI fragment and a 1108 bp ClaI-AccI fragment. A three part ligation was required to replace the native factor VIII sequence with the mutagenized sequence. See figure 9.

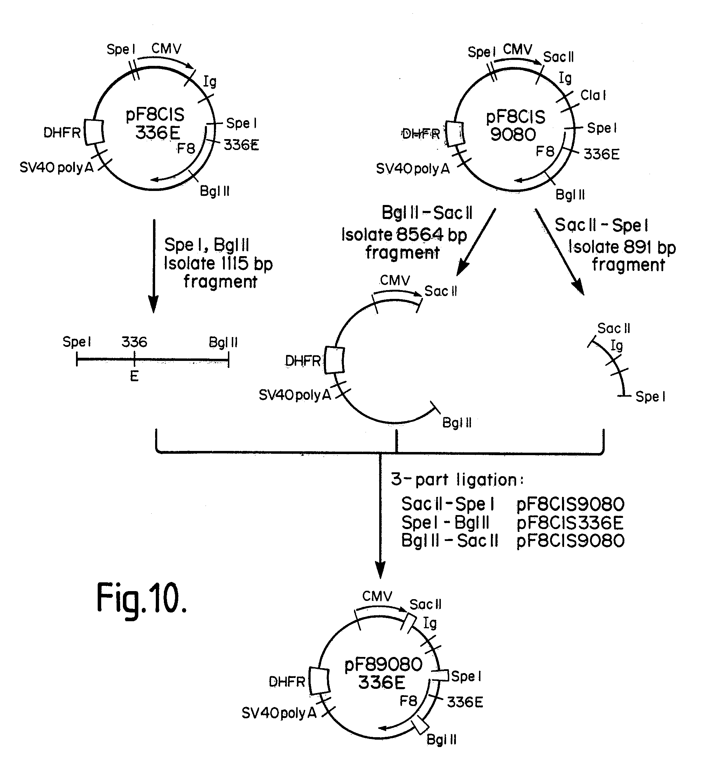

Construction of pF89080-336E proceeded via another three part ligation as shown in Figure 10. A 1115 bp SpeI-BglII fragment containing the 336E variant amino acid was transferred to create another variant fusion protein by ligation to a 891 bp SacII-SpeI fragment and a 8564bp BglII-SacII fragment isolated from pF8CIS9080.

Both of these protein variants were expressed in 293 cells. Full length factor VIII with this mutation was expressed at 2.8 mU/$10^4$ cells/day while the fusion variant was expressed at 15 mU/$10^4$ cells/day.

Example 9

Activity of Factor VIII Resistant to Activated Protein C

Media obtained from 293 cells transfected with pF8CIS-336E shortened the clotting time of hemophilic plasma. This activity was neutralized by a factor VIII polyclonal antibody (Table 3). Activated protein C, however, did not inactivate recombinant factor VIII containing a glutamic acid at position 336 (Table 3).

## Table 3

| Sample | Clot Time | Units/ml |
|---|---|---|
| 293-336E | 70.4 | 0.8 |
| rVIII | 65.8 | 1.1 |
| 293-336E media + APC | 68.5 | 0.9 |
| rVIII + APC | 85.0 | 0.28 |
| 293-336E media Preincubated with factor VIII polyclonal | 95.0 | <0.1 |

Table 3. Stability of full length factor VIII/336E to Activated Protein C. Serum free media that had been conditioned for 48 hrs by transfected 293 cells producing full length factor VIII/336E (referred to as "293-336E" in the table) was concentrated 27 fold. To 100 μl aliquots of this media, 10 μl of rabbit brain cephalin and 10.0 ng of APC was added. Controls received no APC. Samples were incubated for 40 min. at 37°C. Similarly, purified recombinant factor VIII containing the arginine at position 336 (rVIII) was diluted to −1 units/ml with 0.05 M Tris, pH 7.5, 150 mM NaCl, 2.5 mM $CaCl_2$ and incubated with rabbit brain cephalin and APC. Media from transfected 293 cells producing full length factor VIII/336E (26λ) was also preincubated with a factor VIII polyclonal antibody (1μl of IgG prep) for 40 min at 37°C. At the end of incubations, samples were assayed by coagulation analysis.

Example 10

Expression Vector Prorelaxin

1. pCIHRX

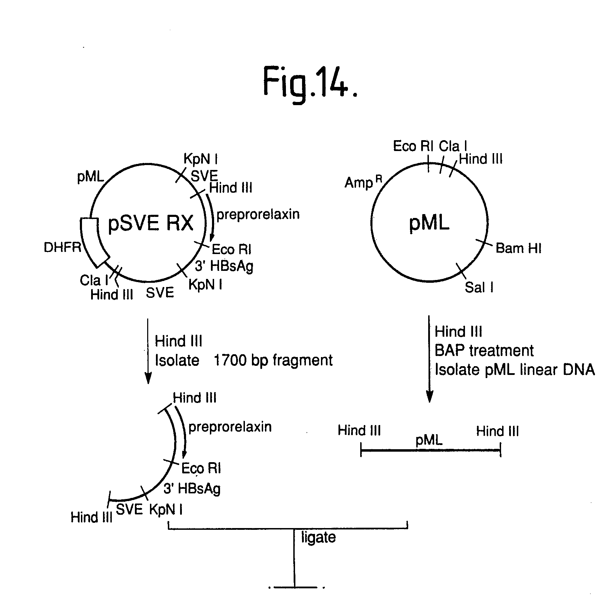

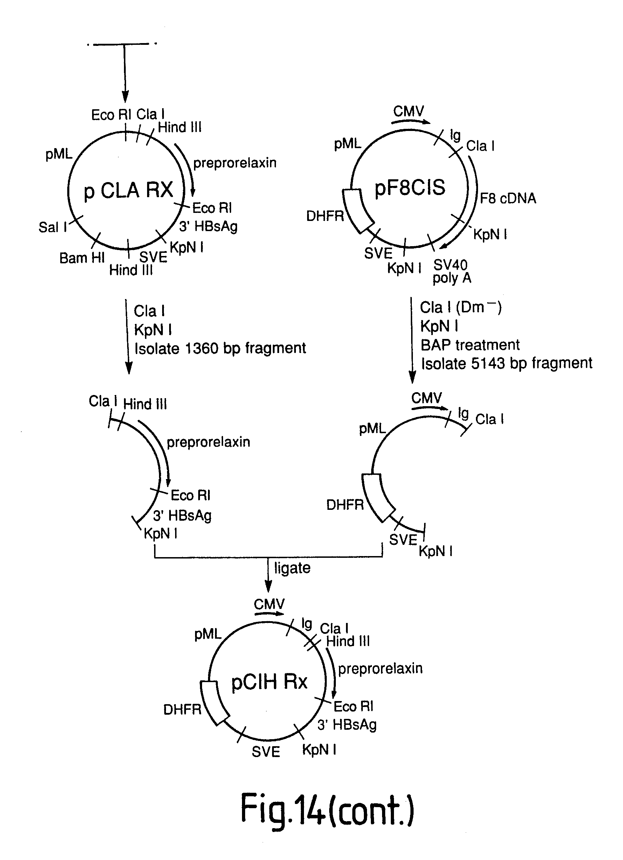

The Vector pCIHRX contained the cytomegalovirus enhancer and promoter, the cytomegalovirus splice donor site, the Ig variable region splice acceptor site, the cDNA encoding H2 preprorelaxin and the hepatitis surface antigen polyadenylation and transcription termination sites. Figure 14 shows the steps for construction of the prorelaxin vector. The same intron and splice acceptor sequence described previously from the Ig variable region was maintained. 677bp of the preprorelaxin cDNA followed these 5′ processing signals. While the 5′ control signals were identical to pF8CIS the polyadenylation region and termination sequence signals were from the hepatitis surface antigen gene rather than SV40.

An intermediate plasmid pClaRX was first constructed The plasmid pSVERX (see copending U.S. patent application U.S.S.N. 06/907,197, filed September 12, 1986, and corresponding European application) was cut with HindIII to isolate a 1700bp fragment containing the pre-prorelaxin cDNA followed by the hepatitis B surface antigen (HBsAg) 3′ polyadenylation site. A KpnI site was 3′ to the HBsAg polyadenylation site and 5′ to the start of the SV40 early promoter which in this vector was used to drive expression of the DHFR cDNA.

This HindIII fragment was inserted into pML linearized at the HindIII site. Reclosures were minimized by treatment with bacterial alkaline phosphatase (BAP). Ampicillin resistant colonies were screened to isolate clones which had inserted the pre-prorelaxin gene so that the 5′ end of the gene was next to the ClaI site of pML.

The intermediate plasmid pCIARX was cut with ClaI and KpnI to isolate a 1360bp fragment containing the pre-prorelaxin gene followed by the hepatitis surface antigen 3′ polyadenylation sequences. This fragment was ligated to the 5143bp fragment created by cutting pF8CIS dam− with ClaI and KpnI.

2. pCISRX

Because the choice of polyadenylation sequences is known to influence 5' processing of messenger RNA (Wilson & Nevins, supra), the 3' hepatitis polyadenylation sequence in pCIHRX was replaced with the pSV40 polyadenylation sequence. This construction was designated pCISRX. The two starting vectors for this construction are pCIHRX and pF8CIS. The latter vector has the same 5' controls as pCIHRX but includes the cDNA for factor VIII and the SV40 polyadenylation site. SacII was used to cleave 3' of the cDNA. The resultant 3' overhang was blunted by T4 polymerase. pCIHRX was then cut with BamHI. This site separates the chimeric intron from the 5' end of the relaxin gene. An 861bp fragment was gel isolated from the BamHI treatment. The SV40 polyadenylation site, DHFR, transcription unit, bacterial origin of replication and amp$^r$ gene, as well as the CMV enhancer and promoter and splice donor were isolated from pF8CIS. These elements were isolated in two fragments, as a 2525bp SalI-BamHI fragment and a HpaI-SalI 3113 bp fragment. A three part ligation of the BamHI-SacII (blunted) fragment with the HpaI- SalI fragment and SalI to BamHI fragment yields pCISRX.

Example 11

Expression Prorelaxin

The expression capabilities of the two relaxin expression vectors pCIHRX and pCISRX, were assayed using several anti-relaxin antibodies in the immunoperoxidase method described above. Three rabbit polyclonals and three mouse monoclonal antibodies were tested on COS cells transfected with pSVERX. One monoclonal RX-I was found to give intense staining with no background.

The two vectors of this invention, pCIHRX and pCISRX, were tested for prorelaxin expression and compared to pSVERX. pCIHRX and pCISRX vectors differed in the polyadenylation sequence. pCIHRX contained the hepatitis surface antigen polyadenylation sequence while pCISRX contained the SV40 early region polyadenylation sequence.

293, TM4 and CHO cells were transfected with 10 μg total DNA which included 1 μg pRSVneo, 5 μg salmon sperm carrier and 4 μg of plasmids pSVERX, pCIHRX and pCISRX. Cells were glycerol shocked as described above. Thirty-six hours following transfection cells were fixed and stained with IH6 to identify transformed cells making prorelaxin. Positive staining cells were seen in 293 and TM4 cells transfected with pCIHRX and pCISRX. Duplicate plates of CHO, 293 and TM4 cells were split and subjected to the staining protocol described above to screen for prorelaxin production cells.

Expression results are shown in the tables below indicating that the vectors containing the stabilizing sequence 5' of the DNA encoding prorelaxin produced significantly higher levels of prorelaxin than the reference plasmid, pSVERX. In the case of stable expression the media assay for prorelaxin was from the general population of cells.

## Transient Expression Prorelaxin

| Cell Type | Plasmid | Amount of Protein (ng/ml) |
|---|---|---|
| CHO | pSVERX | 0.4 |
| | pCIHRX | 0.9 |
| | pCISRX | 3 |
| TM4 | pSVERX | 0.4 |
| | pCIHRX | 2 |
| | pCISRX | 10 |
| 293 | pSVERX | 0.4 |
| | pCIHRX | 3 |
| | pCISRX | 12 |

## Stable Expression Prorelaxin

| Cell Type | Plasmid | Amount of Protein (ng/ml) |
|---|---|---|
| CHO | pSVERX | 0.6 |
| | pCIHRX | 0.8 |
| | pCISRX | 3.9 |
| 293 | pSVERX | 0.41 |
| | pCIHRX | 3.0 |
| | pCISRX | 22.0 |

Example 12

Expression Vector t-PA

1. pCIHt-PA

The vector pCIHt-PA containing the cytomegalovirus enhancer and promoter, the cytomegalovirus splice donor site and intron, the Ig variable region splice acceptor site, the cDNA encoding t-PA (Pennica et al., Nature 301, 214 (1983)) and the hepatitis surface antigen polyadenylation and transcription termination site was constructed.

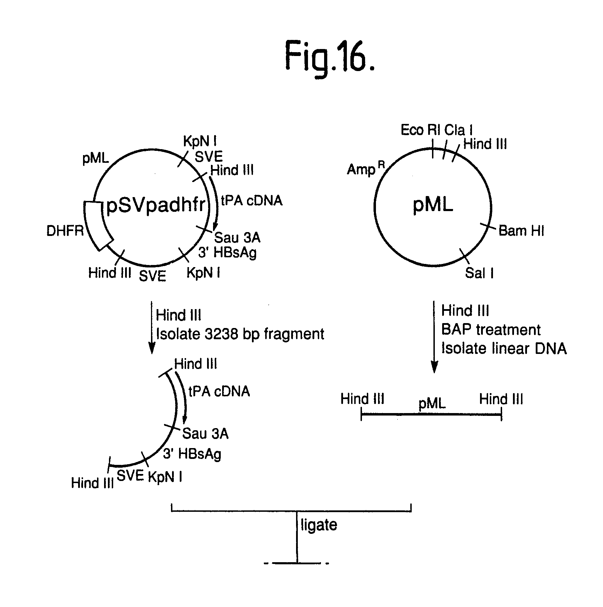

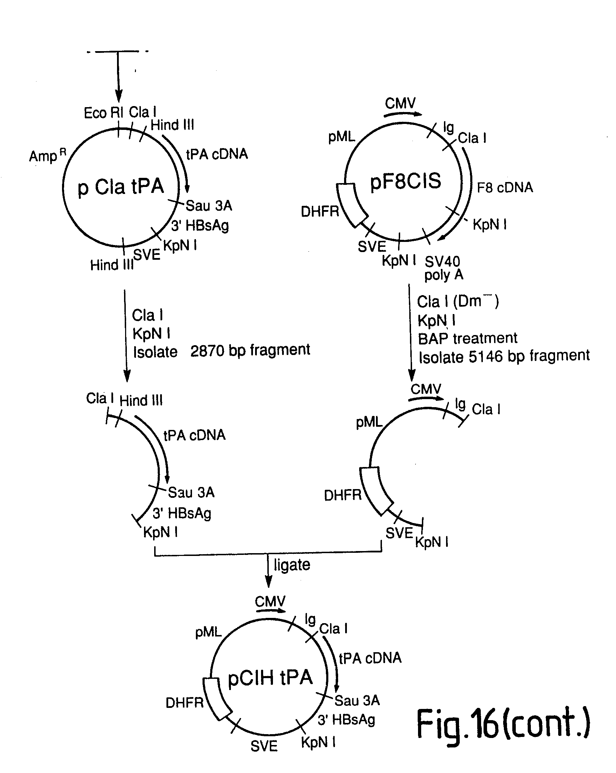

Figure 16 shows the steps for construction of the t-PA vector.

The t-PA cDNA was first cloned into pML to provide a ClaI site at the 5′ end of the gene. To do this a 3238 bp HindIII fragment from pSVpa-DHFR (otherwise referred to as pETPFR in UK patent 2,119,804 B) was inserted into the HindIII site of pML. Colonies were screened for clones which have the 5′ end of the cDNA juxtaposed to the ClaI site. The intermediate plasmid labelled pCLAt-PA is shown in Figure 16. A t-PA cDNA followed by the 3′ polyadenylation region was isolated as a ClaI-KpnI fragment of 2870bp. This fragment was ligated to the 5146bp fragment of pF8CIS. This ClaI-KpnI fragment of the CIS vector provided the 5′ control region, a SV40-DHFR transcriptional unit, the ampicillin resistance gene and origin region from pML. pCIHt-PA is analogous to pCIHRX, discussed above, with the exception of the cDNA coding for the desired heterologous gene.

Expression levels of t-PA were compared by transfecting CHO and 293 cells with pSVpaDHFR, pCMVt-PA and pCIHt-PA. The former two vectors did not contain a stabilizing sequence and thus served as controls for the vector pCIHt-PA containing the cDNA encoding t-PA constructed in accord with the instant invention. Media from each of the cultured transformed 293 cells were assayed and the following results were obtained: pSVpaDHFR gave 30 ng/ml; pCMVt-PA gave 200 ng/ml of t-PA; and pCIHt-PA gave 420 ng/ml of t-PA.

2. pCISt-PA

The vector pCISt-PA containing the cytomegalovirus enhancer and promoter, the cytomegalovirus splice donor site and intron, the Ig variable region splice acceptor site, the cDNA encoding t-PA and the pSV40 polyadenylation sequence was constructed.

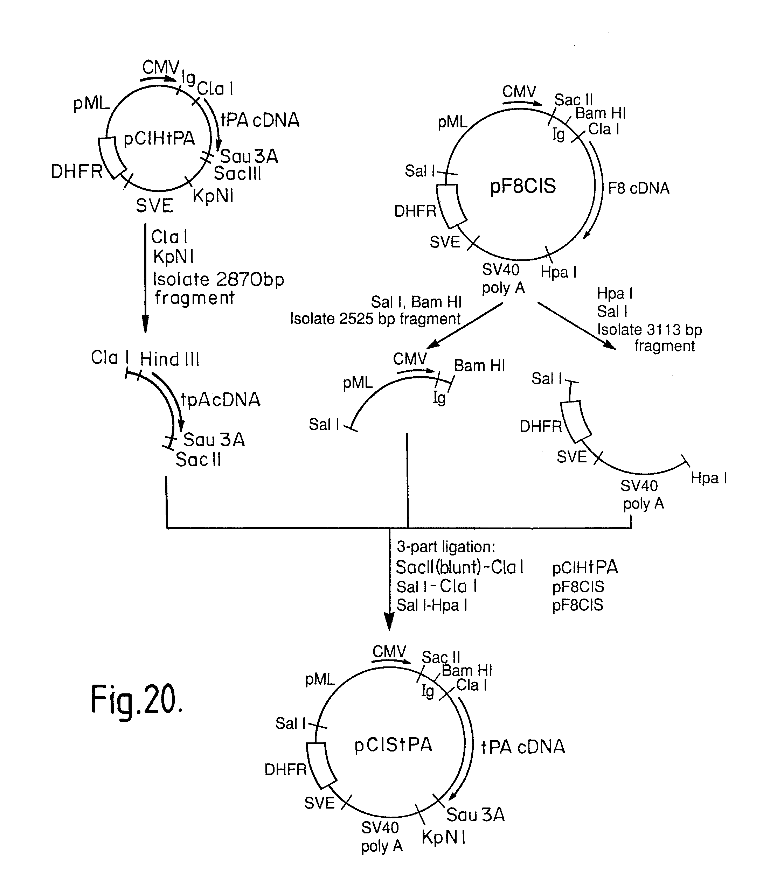

The starting vectors for this construction are pCIHt-PA and pF8CIS (see Figure 20). The latter vector has the same 5' controls as pCIHt-PA but includes the cDNA for factor VIII and the SV40 polyadenylation site. SacII was used to cleave 3' of the t-PA cDNA. The resultant 3' overhang was blunted by T4 polymerase. pCIHt-PA was then cut with ClaI. This site separates the chimeric intron from the 5' end of the t-PA gene. A 2870bp fragment was gel isolated from the ClaI treatment. The SV40 polyadenylation site, DHFR, transcription control, bacterial origin of replication and amp^r gene, as well as the CMV enhancer and promoter and splice donor were isolated from pF8CIS. These elements were isolated into fragments as a 2525bp SalI-ClaI fragment and a HpaI-SalI 3113 fragment. A three part ligation of the SacII(blunt)-ClaI fragment with the HpaI-SalI fragment and SalI-ClaI fragment yields pCISt-PA.

Expression levels of t-PA were compared by transfecting 293 and CHO cells with pCIHt-PA and pCISt-PA. Media from each of the cultured transformed cells were assayed and the following results were obtained:

| | | Transient (t-PA ng/ml) |
|---|---|---|
| CHO | | |
| | CIS | 55 |
| | CIH | 15 |
| 293 | | |
| | CIS | 3000 |
| | CIH | 1300 |

**Claims**

1. A method for continuous production of a desired heterologous protein in a eukaryotic host cell comprising:
   a) constructing an expression vector having a sequence of double stranded DNA comprising the following elements:
   1) a stabilizing sequence downstream of a promoter and upstream of a DNA encoding the amino acid sequence of the desired heterologous protein;
   2) DNA encoding the amino acid sequence of the desired heterologous protein downstream of said stabilizing sequence; and,
   3) DNA coding a polyadenylation sequence downstream of which is a transcription termination site;
   b) transfecting and then choosing a eukaryotic host cell with said expression vector; and
   c) culturing the transfected eukaryotic host cell under conditions favourable for continuous production of the desired protein.

2. The method of claim 1 wherein the promoter is from the immediate early gene of human cytomegalovirus.

3. The method of claim 1 wherein the promoter is from simian virus 40 (SV40).

4. The method of any one of claims 1, 2 and 3 wherein the stabilizing sequence comprises at least one but not more than two splice donor-intron-acceptor sequences.

5. The method of claim 4 wherein the splice donor sequence of the splice donor-intron-acceptor sequence is from the immediate early gene of human cytomegalovirus.

6. The method of claim 4 wherein the intron of the splice donor-intron-acceptor sequence is from the human cytomegalovirus and the immunoglobulin variable region.

7. The method of claim 4 wherein the splice acceptor sequence of the splice donor-intron-acceptor sequence corresponds to the immunoglobulin acceptor sequence.

8. The method of any one of claims 1, 2 and 3 wherein the stabilizing sequence comprises an engineered DNA coding a mRNA having the same properties as a mRNA which had been subject to splicing but from which no nucleotide sequence had in fact been removed.

9. The method of any one of the preceding claims wherein the DNA encoding the amino acid sequence of a heterologous protein encodes factor VIII.

10. The method of any one of claims 1 to 8 wherein the DNA encoding the amino acid sequence of a heterologous protein encodes t-PA.

11. The method of any one of claims 1 to 8 wherein the DNA encoding the amino acid sequence of a heterologous protein encodes prorelaxin.

12. The method of any one of claims 1 to 8 wherein the DNA encoding the amino acid sequence of a heterologous protein encodes a variant of factor VIII.

13. The method of claim 12 wherein the factor VIII is resistant to cleavage by activated protein C.

14. The method of any one of the preceding claims wherein the DNA coding the polyadenylation sequence is from simian virus 40 (SV40).

15. The method of any one of the preceding claims wherein the host cell is mouse sertoli cell (TM4).

16. The method of any one of claims 1 to 14 wherein the host cell is hepatoma cell (HTC).

17. The method of any one of claims 1 to 14 wherein the host cell is human embryonic kidney cell (293).

18. The method of any one of the preceding claims wherein the expression vector includes an enhancer.

19. The method of claim 18 wherein the enhancer is located upstream of the promoter.

20. The method of claim 18 or claim 19 which includes an enhancer and promoter from simian virus (SV40).

21. A vector suitable for continuous expression in a eukaryotic host cell culture of a desired heterologous protein which vector has the features defined in any of the preceding claims.

22. TM4 cells, HTC cells or 293 cells transformed with an expression vector having the features defined in any one of claims 1 to 20.

Fig.1a.

0260148

Fig.1b.

Fig.1c.

Fig.2.

0260148

Fig.3.

0260148

Fig.4.

0260148

Fig.5.

# FIG.6.

Fig.6(cont.)

0260148

Fig.7.

Fig.8.

0260148

Fig.9.

0260148

Fig.10.

## Fig.11.

## Fig.12.

Incubation time (min.)

0260148

# Fig.13.

0260148

# Fig.14.

0260148

Fig.14(cont.)

Fig.15.

# Fig.16.

Fig.16(cont.)

# Fig.17.

```
          aluI
          sacI
          hgiAI
          bsp1286
          banII
          taqI                              speI
      1 TTCGAGCTCG CCCGACATTG ATTATTGACT AGTTATTAAT AGTAATCAAT TACGGGGTCA
        AAGCTCGAGC GGGCTGTAAC TAATAACTGA TCAATAATTA TCATTAGTTA ATGCCCCAGT
        from pPMLCMV beginning to HindIII,enhancers and promoter


                                                                       scrFI
                                                                 bglI bstNI
                                                                 sau96I
                                    thaI                         haeIII
     61 TTAGTTCATA GCCCATATAT GGAGTTCCGC GTTACATAAC TTACGGTAAA TGGCCCGCCT
        AATCAAGTAT CGGGTATATA CCTCAAGGCG CAATGTATTG AATGCCATTT ACCGGGCGGA


                              ahaII
                              aatII
    121 GGCTGACCGC CCAACGACCC CCGCCCATTG ACGTCAATAA TGACGTATGT TCCCATAGTA
        CCGACTGGCG GGTTGCTGGG GGCGGGTAAC TGCAGTTATT ACTGCATACA AGGGTATCAT


                              ahaII
                              aatII                              bglI
    181 ACGCCAATAG GGACTTTCCA TTGACGTCAA TGGGTGGAGT ATTTACGGTA AACTGCCCAC
        TGCGGTTATC CCTGAAAGGT AACTGCAGTT ACCCACCTCA TAAATGCCAT TTGACGGGTG


                                                           ahaII
          rsaI                 ndeI       rsaI             aatII
    241 TTGGCAGTAC ATCAAGTGTA TCATATGCCA AGTACGCCCC CTATTGACGT CAATGACGGT
        AACCGTCATG TAGTTCACAT AGTATACGGT TCATGCGGGG GATAACTGCA GTTACTGCCA


          bglI
          sau96I scrFI                   nlaIII
          haeIII bstNI        rsaI                                     rsaI
    301 AAATGGCCCG CCTGGCATTA TGCCCAGTAC ATGACCTTAT GGGACTTTCC TACTTGGCAG
        TTTACCGGGC GGACCGTAAT ACGGGTCATG TACTGGAATA CCCTGAAAGG ATGAACCGTC


                              nlaIII
                              styI     sfaNI
          snaBI               ncoI hphI                    rsaI
    361 TACATCTACG TATTAGTCAT CGCTATTACC ATGGTGATGC GGTTTTGGCA GTACATCAAT
        ATGTAGATGC ATAATCAGTA GCGATAATGG TACCACTACG CCAAAACCGT CATGTAGTTA


                                                           ahaII
                         hinfI                             aatII
    421 GGGCGTGGAT AGCGGTTTGA CTCACGGGGA TTTCCAAGTC TCCACCCCAT TGACGTCAAT
        CCCGCACCTA TCGCCAAACT GAGTGCCCCT AAAGGTTCAG AGGTGGGGTA ACTGCAGTTA


                         nlaIV
                         banI
    481 GGGAGTTTGT TTTGGCACCA AAATCAACGG GACTTTCCAA AATGTCGTAA CAACTCCGCC
        CCCTCAAACA AAACCGTGGT TTTAGTTGCC CTGAAAGGTT TTACAGCATT GTTGAGGCGG
```

```
                                                                    aluI
                                                                    sacI
                                                                    hgiAI
                                                                    bsp1286
          hgaI                           rsaI      mnlI            banII
541 CCATTGACGC AAATGGGCGG TAGGCGTGTA CGGTGGGAGG TCTATATAAG CAGAGCTCGT
    GGTAACTGCG TTTACCCGCC ATCCGCACAT GCCACCCTCC AGATATATTC GTCTCGAGCA


                      scrFI
                      sau3AI        hgaI
                      dpnI bstNI    ahaII fokI                mnlI      mboII
601 TTAGTGAACC GTCAGATCGC CTGGAGACGC CATCCACGCT GTTTTGACCT CCATAGAAGA
    AATCACTTGG CAGTCTAGCG GACCTCTGCG GTAGGTGCGA CAAAACTGGA GGTATCTTCT
                      Begin RNA


                                          scrFI
          sau96I                          nciI
           avaII                          haeIII
           nlaIV                          xmaIII
          scrFI                           eaeI
          nciI                            fnu4HI
          mspI       sau3AI mnlI thaI mspI
          hpaII      dpnI  bglI sacII hpaII                 thaI hinfI
661 CACCGGGACC GATCCAGCCT CCGCGGCCGG GAACGGTGCA TTGGAACGCG GATTCCCCGT
    GTGGCCCTGG CTAGGTCGGA GGCGCCGGCC CTTGCCACGT AACCTTGCGC CTAAGGGGCA


                                                  bstXI
                                                  sau96I
                      rsaI          hinfI      haeIII     styI
721 GCCAAGAGTG ACGTAAGTAC CGCCTATAGA GTCTATAGGC CCACCCCCTT GGCTTCTTAT
    CGGTTCTCAC TGCATTCATG GCGGATATCT CAGATATCCG GGTGGGGGAA CCGAAGAATA


                                                  haeI
                                                  eaeI
          sau3AI                                  balI
          dpnI                                    sau3AI
          xhoII           aluI                    dpnI
          nlaIV         ddeI      mnlI            xhoII
          bamHI       rsaI hindIII                bglII haeIII
781 GCGACGGATC CCGTACTAAG CTTGAGGTGT GGCAGGCTTG AGATCTGGCC ATACACTTGA
    CGCTGCCTAG GGCATGATTC GAACTCCACA CCGTCCGAAC TCTAGACCGG TATGTGAACT
                      IgE synthetic 100mer


                                                                    fnu4HI
                                                                    bbvI
                      fokI                                          pstI
841 GTGACAATGA CATCCACTTT GCCTTTCTCT CCACAGGTGT CCACTCCCAC GTCCAACTGC
    CACTGTTACT GTAGGTGAAA CGGAAAGAGA GGTGTCCACA GGTGAGGGTG CAGGTTGACG
                                                                   PstI-ClaI
                                                                   converter


                      claI
                      sau3AI
                      dpnI
                      pvuI
          aluI      taqI taqI
901 AGCTCGGTTC GATCGATAA
    TCGAGCCAAG CTAGCTATT
```

Fig.17(cont.)

# Fig.18.

```
                                                        nlaIV
                                                        scrFI
  xmnI                                                  bstNI
  1 GAATTAATTC TGTGGAATGT GTGTCAGTTA GGGTGTGGAA AGTCCCCAGG CTCCCCAGCA
    CTTAATTAAG ACACCTTACA CACAGTCAAT CCCACACCTT TCAGGGGTCC GAGGGGTCGT

                   nsiI
                   avaIII
                   nlaIII                           scrFI              scrFI
                   sphI sfaNI                       bstNI              bstNI
 61 GGCAGAAGTA TGCAAAGCAT GCATCTCAAT TAGTCAGCAA CCAGGTGTGG AAAGTCCCCA
    CCGTCTTCAT ACGTTTCGTA CGTAGAGTTA ATCAGTCGTT GGTCCACACC TTTCAGGGGT

                             nsiI
                             avaIII
                             nlaIII
  nlaIV                      sphI sfaNI
121 GGCTCCCCAG CAGGCAGAAG TATGCAAAGC ATGCATCTCA ATTAGTCAGC AACCATAGTC
    CCGAGGGGTC GTCCGTCTTC ATACGTTTCG TACGTAGAGT TAATCAGTCG TTGGTATCAG

                                                                  styI
                 fokI                                             ncoI
181 CCGCCCCTAA CTCCGCCCAT CCCGCCCCTA ACTCCGCCCA GTTCCGCCCA TTCTCCGCCC
    GGCGGGGATT GAGGCGGGTA GGGCGGGGAT TGAGGCGGGT CAAGGCGGGT AAGAGGCGGG

                                      fnu4HI
                                      sfiI
                                      haeIII              ddeI
                                 haeIII bglI       haeIII
  nlaIII                         mnlI  mnlI     mnlI  mnlI    aluI
241 CATGGCTGAC TAATTTTTTT TATTTATGCA GAGGCCGAGG CCGCCTCGGC CTCTGAGCTA
    GTACCGACTG ATTAAAAAAA ATAAATACGT CTCCGGCTCC GGCGGAGCCG GAGACTCGAT

                                      styI
                                      haeIII
                                      stuI
                       mnlI          haeI
             mnlI                    mnlI  avrII                aluI ecoRI sau96I
301 TTCCAGAAGT AGTGAGGAGG CTTTTTTGGA GGCCTAGGCT TTTGCAAAAA GCTGAATTCG
    AAGGTCTTCA TCACTCCTCC GAAAAAACCT CCGGATCCGA AAACGTTTTT CGACTTAAGC
```

0260148

```
     scrFI
     nciI
     mspI
     hpaII                                  hinfI
     haeIII                       thaI                                          rsaI
361  GGCCGGGAAC GGTGCATTGG AACGCGGATT CCCCGTGCCA AGAGTGACGT AAGTACCGCC
     CCGGCCCTTG CCACGTAACC TTGCGCCTAA GGGGCACGGT TCTCACTGCA TTCATGGCGG
     SacI site from pAML3P
     SacII site from CMVS


                                                      sau3AI
                                                      dpnI
                              bstXI                   xhoII          aluI
                         sau96I                       nlaIV     ddeI    mnlI
          hinfI          haeIII      styI             bamHI rsaI hindIII
421  TATAGAGTCT ATAGGCCCAC CCCCTTGGCT TCTTATGCGA CGGATCCCGT ACTAAGCTTG
     ATATCTCAGA TATCCGGGTG GGGGAACCGA AGAATACGCT GCCTAGGGCA TGATTCGAAC
                                                                 IgE synthetic
                                                                 100mer


                                   haeIII
                         sau3AI
                         dpnI haeI
                         xhoII eaeI
                         bglII balI                            fokI
481  AGGTGTGGCA GGCTTGAGAT CTGGCCATAC ACTTGAGTGA CAATGACATC CACTTTGCCT
     TCCACACCGT CCGAACTCTA GACCGGTATG TGAACTCACT GTTACTGTAG GTGAAACGGA


                                                          taqI
                                                          claI
                                          aluI            sau3AI
                                          fnu4HI          dpnI
                                          bbvI            pvuI
                                          pstI            taqI
541  TTCTCTCCAC AGGTGTCCAC TCCCACGTCC AACTGCAGCT CGGTTCGATC GATAA
     AAGAGAGGTG TCCACAGGTG AGGGTGCAGG TTGACGTCGA GCCAAGCTAG CTATT
                                          PstI-ClaI converter
```

# Fig.18(cont.)

# Fig.19.

```
     aluI
     sacI
     hgiAI
     bsp1286
     banII
     taqI                                    speI
   1 TTCGAGCTCG CCCGACATTG ATTATTGACT AGTTATTAAT AGTAATCAAT TACGGGGTCA
     AAGCTCGAGC GGGCTGTAAC TAATAACTGA TCAATAATTA TCATTAGTTA ATGCCCCAGT
     from pPMLCMV beginning to HindIII,enhancers and promoter


                                                                   scrFI
                                                            bglI bstNI
                                                            sau96I
                                           thaI              haeIII
  61 TTAGTTCATA GCCCATATAT GGAGTTCCGC GTTACATAAC TTACGGTAAA TGGCCCGCCT
     AATCAAGTAT CGGGTATATA CCTCAAGGCG CAATGTATTG AATGCCATTT ACCGGGCGGA


                                           ahaII
                                           aatII
 121 GGCTGACCGC CCAACGACCC CCGCCCATTG ACGTCAATAA TGACGTATGT TCCCATAGTA
     CCGACTGGCG GGTTGCTGGG GGCGGGTAAC TGCAGTTATT ACTGCATACA AGGGTATCAT


                              ahaII
                              aatII                              bglI
 181 ACGCCAATAG GGACTTTCCA TTGACGTCAA TGGGTGGAGT ATTTACGGTA AACTGCCCAC
     TGCGGTTATC CCTGAAAGGT AACTGCAGTT ACCCACCTCA TAAATGCCAT TTGACGGGTG


                                                        ahaII
          rsaI                ndeI      rsaI            aatII
 241 TTGGCAGTAC ATCAAGTGTA TCATATGCCA AGTACGCCCC CTATTGACGT CAATGACGGT
     AACCGTCATG TAGTTCACAT AGTATACGGT TCATGCGGGG GATAACTGCA GTTACTGCCA


          bglI
         'sau96I scrFI                    nlaIII
          haeIII bstNI          rsaI                           rsaI
 301 AAATGGCCCG CCTGGCATTA TGCCCAGTAC ATGACCTTAT GGGACTTTCC TACTTGGCAG
     TTTACCGGGC GGACCGTAAT ACGGGTCATG TACTGGAATA CCCTGAAAGG ATGAACCGTC


                              nlaIII
                              styI      sfaNI
          snaBI               ncoI hphI                 rsaI
 361 TACATCTACG TATTAGTCAT CGCTATTACC ATGGTGATGC GGTTTTGGCA GTACATCAAT
     ATGTAGATGC ATAATCAGTA GCGATAATGG TACCACTACG CCAAAACCGT CATGTAGTTA
```

```
                                                                 ahaII
                         hinfI                                   aatII
421 GGGCGTGGAT AGCGGTTTGA CTCACGGGGA TTTCCAAGTC TCCACCCCAT TGACGTCAAT
    CCCGCACCTA TCGCCAAACT GAGTGCCCCT AAAGGTTCAG AGGTGGGGTA ACTGCAGTTA


                         nlaIV
                         banI
481 GGGAGTTTGT TTTGGCACCA AAATCAACGG GACTTTCCAA AATGTCGTAA CAACTCCGCC
    CCCTCAAACA AAACCGTGGT TTTAGTTGCC CTGAAAGGTT TTACAGCATT GTTGAGGCGG


                                                                 aluI
                                                                 sacI
                                                                 hgiAI
                                                                 bsp1286
          hgaI                      rsaI       mnlI              banII
541 CCATTGACGC AAATGGGCGG TAGGCGTGTA CGGTGGGAGG TCTATATAAG CAGAGCTCGT
    GGTAACTGCG TTTACCCGCC ATCCGCACAT GCCACCCTCC AGATATATTC GTCTCGAGCA


                      scrFI
          sau3AI          hgaI
          dpnI  bstNI  ahaII fokI                  mnlI       mboII
601 TTAGTGAACC GTCAGATCGC CTGGAGACGC CATCCACGCT GTTTTGACCT CCATAGAAGA
    AATCACTTGG CAGTCTAGCG GACCTCTGCG GTAGGTGCGA CAAAACTGGA GGTATCTTCT
                Begin RNA


                              scrFI
          sau96I              nciI
          avaII               haeIII
          nlaIV               xmaIII
          scrFI               eaeI
          nciI                fnu4HI
          mspI     sau3AI  mnlI thaI mspI
          hpaII    dpnI    bglI sacII hpaII    hphI           thaI hinfI
661 CACCGGGACC GATCCCAGCC TCCGCGGCCG GGAACGGTGA TTGGAACGCG GATTCCCCGT
    GTGGCCCTGG CTAGGGTCGG AGGCGCCGGC CCTTGCCACT AACCTTGCGC CTAAGGGGCA


                                                        claI
                                        aluI            sau3AI
                                        fnu4HI          dpnI
                                        bbvI     mspI taqI taqI
                   tth111I              pstI     hpaII pvuI
721 GCCAAGAGTG ACGGTGTCCA CTCCCACGTC CAACTGCAGC TCCGGTTCGA TCGATAA
    CGGTTCTCAC TGCCACAGGT GAGGGTGCAG GTTGACGTCG AGGCCAAGCT AGCTATT
```

## Fig.19(cont.)

0260148

Fig.20.